# EUROPEAN PATENT SPECIFICATION

(11) **EP 2 059 198 B1**
(45) Date of publication and mention of the grant of the patent: **15.01.2014**
(21) Application number: 07837762.9
(22) Date of filing: 06.09.2007
(51) Int. Cl.: A61F 2/89, A61F 2/91, A61F 2/95, A61F 2/915

(54) **STENTS WITH CONNECTORS AND STABILIZING BIODEGRADABLE ELEMENTS**
STENTS MIT STECKVERBINDERN UND BIOLOGISCH ABBAUBAREN STABILISIERUNGSELEMENTEN
STENTS AVEC RACCORDS ET ÉLÉMENTS DE STABILISATION BIODÉGRADABLES

(30) Priority: 06.09.2006 US 842475 P; 02.11.2006 US 856658 P
(43) Date of publication of application: 20.05.2009
(60) Divisional application: 11173093.3
(73) Proprietor: Cook Medical Technologies LLC, Bloomington, IN 47404 (US)
(72) Inventor: LEEWOOD, Alan R., Lafayette, IN 47905 (US); GREWE, David D., West Lafayette, IN 47906 (US); HIATT, Mark, Elletsville Indiana 47429 (US); RAGHEB, Anthony O., West Lafayette, IN 47906 (US); VOORHEES, III, William D., West Lafayette, IN 47906 (US); ROEDER, Blayne A., Lafayette, IN 47909 (US); PURDY, James D., Lafayette, IN 47904 (US)
(74) Representative: Garratt, Peter Douglas
(86) International application number: PCT/US2007/019389
(87) International publication number: WO 2008/030488

(56) References cited:
- EP-A- 1 173 109
- EP-A- 1 563 806
- WO-A-2006/029617
- WO-A-2006/060534
- US-A1- 2005 125 051
- US-A1- 2006 122 691

## Description

### Related Applications

This application claims the benefit of priority from U.S. Provisional Application No. 60/856,658, filed November 2, 2006 and from 60/842,475, filed September 6, 2006.

### Technical Field

The present invention relates generally to medical devices and more particularly to intraluminal devices.

### Background of the Invention

Stents have become relatively common devices for treating a number of organs, such as the vascular system, colon, biliary tract, urinary tract, esophagus, trachea and the like. Stents are useful in treating various ailments including blockages, occlusions, narrowing conditions and other related problems that restrict flow through a passageway (generally referred to as a stenosis). Stents are also useful in a variety of other medical procedures including treating various types of aneurysms.

For example, stents may be used to treat numerous vessels in the vascular system, including coronary arteries, peripheral arteries (e.g., carotid, brachial, renal, iliac and femoral), and other vessels. Stents have become a common alternative for treating vascular conditions because stenting procedures are considerably less invasive than other alternatives. As an example, stenoses in the coronary arteries have traditionally been treated with bypass surgery. In general, bypass surgery involves splitting the chest bone to open the chest cavity and grafting a replacement vessel onto the heart to bypass the stenosed artery. However, coronary bypass surgery is a very invasive procedure that is risky and requires a long recovery time for the patient. By contrast, stenting procedures are performed transluminal^ and do not require open surgery. Thus, recovery time is reduced and the risks of surgery are minimized.

Many different types of stents and stenting procedures are possible. In general, however, stents are typically designed as tubular support structures that may be inserted percutaneously and transluminally through a body passageway. Typically, stents are made from a structure that wraps around at least a portion of a circumference and are adapted to compress and expand between a smaller and larger diameter. However, other types of stents are designed to have a fixed diameter and are not generally compressible. Although stents may be made from many types of materials, including non-metallic materials and natural tissues, common examples of metallic materials that may be used to make stents include stainless steel and Nitinol. Other materials may also be used, such as cobaltchrome alloys, amorphous metals, tantalum, platinum, gold, titanium, polymers and/or compatible tissues. Typically, stents are implanted within an artery or other passageway by positioning the stent within the lumen to be treated and then expanding the stent from a compressed diameter to an expanded diameter. The ability of the stent to expand from a compressed diameter makes it possible to thread the stent through narrow, tortuous passageways to the area to be treated while the stent is in a relatively small, compressed diameter. Once the stent has been positioned and expanded at the area to be treated, the tubular support structure of the stent contacts and radially supports the inner wall of the passageway. The implanted stent may be used to mechanically prevent the passageway from closing in order to keep the passageway open to facilitate fluid flow through the passageway. Stents may also be used to support a graft layer. However, these are only some of the examples of how stents may be used, and stents may be used for other purposes as well.

Examples of known stent structures may be found in US 2006/122691A1, which discloses a stent provided with a series of short pieces or sections connected together by a tube made of bioresorbable polymer. The continuous covering of the tubing may inhibit embolization in the first few weeks after stent implantation within the walls of a vessel and timing for removal of the tube through formulation of the bioresorbable polymer can be controlled to occur when embolization is no longer a risk. The stent sections are designed to separate or articulate with time as the polymer biodegrades. The time of separation can be controlled by the characteristics of the bioresorbable polymer to allow the stent to be buried in neo-intima. When the detachment of the stent pieces or sections occurs, they are fixedly secured within the vessel and each is able to flex with the vessel independently of the other stent segments.

Stents may also be used in combination with other components to treat a number of medical conditions. For example, stent-graft assemblies are commonly used in the treatment of aneurysms. As those in the art well know, an aneurysm is an abnormal widening or ballooning of a portion of an artery. Generally, this condition is caused by a weakness in the blood vessel wall. High blood pressure and atherosclerotic disease may also contribute to the formation of aneurysms. Common types of aneurysms include aortic aneurysms, cerebral aneurysms, popliteal artery aneurysms, mesenteric artery aneurysms, and splenic artery aneurysms. However, it is also possible for aneurysms to form in blood vessels throughout the vasculature. If not treated, an aneurysm may eventually rupture, resulting in internal hemorrhaging. In many cases, the internal bleeding may be so massive that a patient might die within minutes of an aneurysm rupture. For example, in the case of aortic aneurysms, the survival rate after a rupture can be as low as 20%.

Traditionally, aneurysms have been treated with surgery. For example, in the case of an abdominal aortic aneurysm, the abdomen is surgically opened, and the widened section of the aorta is typically dissected longitudinally. A graft material, such as Dacron, is then inserted into the vessel and sutured at each end to the inner wall of the non-widened portions of the vessel. The dissected edges of the vessel may then be overlapped and sutured to enclose the graft material within the vessel. In smaller vessels where the aneurysm forms a balloon-like bulge with a narrow neck connecting the aneurysm to the vessel, the surgeon may put a clip on the blood vessel wall at the neck of the aneurysm between the aneurysm and the primary passageway of the vessel. The clip then prevents blood flow from the vessel from entering the aneurysm.

An alternative to traditional surgery is endovascular treatment of the blood vessel with a stent-graft. This alternative involves implanting a stent-graft in the blood vessel across the aneurysm using conventional catheter-based placement techniques. The stent-graft treats the aneurysm by sealing the wall of the blood vessel with a generally impermeable graft material. Thus, the aneurysm is sealed off and blood flow is kept within the primary passageway of the blood vessel. Increasingly, treatments using stent-grafts are becoming preferred since the procedure results in less trauma and a faster recuperation.

Particular stent designs and implantation procedures vary widely. For example, stents are often generally characterized as either balloon-expandable or self-expanding. However, the uses for balloon-expandable and self-expanding stents may overlap and procedures related to one type of stent are sometimes adapted to other types of stents.

Balloon-expandable stents are frequently used to treat stenosis of the coronary arteries. Usually, balloon-expandable stents are made from ductile materials that plastically deform relatively easily. In the case of stents made from metal, 316L stainless steel which has been annealed is a common choice for this type of stent. One procedure for implanting balloon-expandable stents involves mounting the stent circumferentially on the balloon of a balloon-tipped catheter and threading the catheter through a vessel passageway to the area to be treated. Once the balloon is positioned at the narrowed portion of the vessel to be treated, the balloon is expanded by pumping saline, along with contrast solution if desired, through the catheter to the balloon. The balloon then simultaneously dilates the vessel and radially expands the stent within the dilated portion. The balloon is then deflated and the balloon-tipped catheter is retracted from the passageway. This leaves the expanded stent permanently implanted at the desired location. Ductile metal lends itself to this type of stent since the stent may be compressed by plastic deformation to a small diameter when mounted onto the balloon. When the balloon is later expanded in the vessel, the stent once again plastically deforms to a larger diameter to provide the desired radial support structure. Traditionally, balloon-expandable stents have been more commonly used in coronary vessels than in peripheral vessels because of the deformable nature of these stents. One reason for this is that peripheral vessels tend to experience frequent traumas from external sources (e.g., impacts to a person's arms, legs, etc.) which are transmitted through the body's tissues to the vessel. In the case of peripheral vessels, there is an increased risk that an external trauma could cause a balloon-expandable stent to once again plastically deform in unexpected ways with potentially severe and/or catastrophic results. In the case of coronary vessels, however, this risk is minimal since coronary vessels rarely experience traumas transmitted from external sources. In addition, one advantage of balloon-expandable stents is that the expanded diameter of the stent may be precisely controlled during implantation. This is possible because the pressure applied to the balloon may be controlled by the physician to produce a precise amount of radial expansion and plastic deformation of the stent. Another advantage of balloon-expandable stents is that it may be easier to precisely implant the stent at the longitudinal position of the treatment site.

Self-expanding stents are increasingly being used by physicians because of their adaptability to a variety of different conditions and procedures. Self-expanding stents are usually made of shape memory materials or other elastic materials that act like a spring. Typical materials used in this type of stent include Nitinol, 304 stainless steel, and certain polymers. However, other materials may also be used. To facilitate stent implantation, self-expanding stents are normally installed on the end of a catheter in a low profile, compressed state. The stent is typically retained in the compressed state by inserting the stent into a sheath at the end of the catheter. The stent is then guided to the portion of the vessel to be treated. Once the catheter and stent are positioned adjacent to the portion to be treated, the stent is released by pulling, or withdrawing, the sheath rearward. Normally, a step or other feature is provided on the catheter to prevent the stent from moving rearward with the sheath. After the stent is released from the retaining sheath, the stent springs radially outward to an expanded diameter until the stent contacts and presses against the vessel wall. Traditionally, self-expanding stents have been used in a number of peripheral arteries in the vascular system due to the elastic characteristic of these stents. One advantage of self-expanding stents for peripheral arteries is that traumas from external sources do not permanently deform the stent. As a result, the stent may temporarily deform during unusually harsh traumas and spring back to its expanded state once the trauma is relieved. However, self-expanding stents may be used in many other applications as well.

One particularly challenging body passageway to treat is the superficial femoral artery (SFA), which extends along the thigh and passes through the knee. As a person walks and moves about, the SFA experiences substantial changes in shape. For example, knee movement may cause the SFA to undergo axial compression, thereby causing the axial length portions of the SFA to change in length by as much as 20%. The SFA also undergoes significant axial bending, thereby causing unstented portions of the SFA to wrinkle and kink. Because of these characteristics, it has been difficult to design stents that conform adequately to the SFA and maintain patency, In addition, the fatigue life of a stent may be reduced due to the continuous movement of the SFA.

Various alternatives have been considered for the treatment of the SFA. For example, multiple stents may be used to treat the SFA, with the ends of adjacent stents overlapping each other. This alternative ensures complete treatment of a portion of the SFA and allows some movement between the stents. Multiple stents may also be implanted with gaps between the stents. This alternative allows greater movement between adjacent stents but leaves the gaps between the stents unsupported and allows restenosis to potentially occur within the gaps. Furthermore, the stresses and strains from the continuous movement of the SFA may cause multiple implanted stents, if originally separated, to touch and become intertwined with each other. The intertwining of the struts may also result in fracture or eventual breakage of the stent struts. Broken struts can penetrate into the artery wall. Current perception is that this penetration leads to partial or total stenosis at the penetration site.

A single long stent may also be used. However, it is difficult to design a single long stent which has sufficient flexibility and fatigue life. Accordingly, conventional stents usually do not perform adequately when implanted in vessels such as the SFA.

Additionally, the actual number of stents required to be deployed is not always apparent at the start of the medical procedure. For example, after deploying a stent at a first target site, an arteriogram may indicate that another target site requires implantation of a stent. At this stage in the medical procedure, the withdrawal and insertion of a new delivery device may dislodge or disrupt the previously implanted stents. Furthermore, implantation of a first stent may have caused a tissue tear near the vicinity of the stent because it over expanded.

The above-described examples are only some of the applications in which stents are used by physicians. Many other applications for stents are known and/or may be developed in the future.

### Summary of the invention

A stent is described which may be used to treat the superficial femoral artery (SFA). The stent includes a stent structure made of non-biodegradable interconnecting members. Biodegradable connectors are connected to the interconnecting members. The stent may be particularly useful in treating the SFA because the stent structure may be provided with fewer non-biodegradable interconnecting members to improve flexibility and fatigue properties of the support structure. However, the biodegradable connectors stabilize the support structure of the stent during deployment so that the stent deploys uniformly. The multiple stent design comprises expandable stent segments configured in series and which are interconnected by biodegradable interconnectors that span between adjacent stent segments. The stent segments form a generally tubular structure with a longitudinal axis that is coaxial with each of the longitudinal axes of the stent segments. After the stent is deployed, the biodegradable connectors degrade or are absorbed. As a result, only the non-biodegradable support structure remains. Additional details and advantages are described below in the detailed description.

Thus, in accordance with a first aspect of the present invention there is provided a medical device configured for delivery along a body lumen and implantation at a delivery site, the device comprising a plurality of respective stents arranged along a common axis, each stent comprising a plurality of ring-shaped structures and having a radially compressed form for delivery and a radially expanded form, there being provided at least one temporary stabilizing element shaped as a polymer strip which is aligned along the longitudinal direction of the stents and operatively interconnects at least two of the structures to reduce the axial flexibility of the medical device during delivery and implantation, the stabilizing element being adapted to be disconnected after delivery and implantation to increase the flexibility of the device post implantation.

In accordance with a second method of manufacturing a medical device comprising a plurality of respective stents arranged along a common axis with stabilizing elements, comprising the steps of inserting a plurality of expandable stents into a transfer tube, the transfer tube comprising one or more slots; expanding the stents partially within the slotted transfer tube; spraying a semi-rigid or rigid polymeric material through the one or more slots onto an outer surface of a plurality of struts of the stents; and curing the sprayed outer surface.

Additionally, the following constructions may assist in understanding the present invention.

A multiple stent structure, comprising: a plurality of separate expandable stent segments configured in linear order, the stent segments forming a generally tubular structure with a longitudinal axis, the longitudinal axis of the tubular structure being coaxial with longitudinal axes of the stent segments; a sleeve affixed to the plurality of stent segments and extending from a proximal end of the generally tubular structure to a distal end of the generally tubular structure, wherein the plurality of stent segments are connected to each other by the sleeve; and a plurality of openings extending through the sleeve.

The multiple stent structure, wherein a lubricious coating overlies a surface of the sleeve to lower the coefficient of friction between the surface and an outer sheath.

The multiple stent structure, wherein the lubricious coating comprises a hydrophilic polymer or hydrogel.

The multiple stent structure, wherein the sleeve comprises expanded polytetrafluoroethylene (ePTFE).

The multiple stent structure, wherein the plurality of openings comprise between about 5% to about 80% of an overall circumferential surface area of the stent structure.

The multiple stent structure, wherein the plurality of openings comprise between about 35% to about 65% of an overall circumferential surface area of the stent structure.

The multiple stent structure, wherein the plurality of openings are generally circular shaped.

The multiple stent structure, wherein the sleeve comprises a non-biodegradable material.

The multiple stent structure, wherein the non-biodegradable material is a polyetherurethaneurea blended with a surface modifying siloxane-based additive.

A multiple stent structure, comprising:
a plurality of expandable stent segments configured in linear order within an outer sheath, the stent segments forming a generally tubular structure with a longitudinal axis, the longitudinal axis of the tubular structure being coaxial with longitudinal axes of the stent segments, a first stent segment of the plurality of stent segments having a first proximal end portion and a first distal end portion, a second stent segment of the plurality of stent segments having a second proximal end portion and a second distal end portion, the second proximal end portion being adjacent to the first distal end portion;
a first biodegradable interconnector element attached between the first distal end portion and the second proximal end portion; and
a first spacer element affixed to an outer surface of an inner catheter, the first spacer element situated between the first and the second stent segments, wherein the first spacer element has a first depth sufficient to prevent contact of the first and second stent segments during deployment when the outer sheath is positioned proximal relative to the inner catheter.

The multiple stent structure, wherein endothelial growth-promoting agents are disposed on an inner surface of at least one of the plurality of stent segments.

The multiple stent structure, wherein the first biodegradable interconnector element prevents contact of the first distal end portion and the second proximal end portion within a body lumen.

The multiple stent structure, wherein the first spacer element comprises a plurality of first segmented portions, wherein each of the plurality of first segmented portions extending only partially around a circumference of the first distal end portion and the second proximal end portion.

The multiple stent structure, wherein the first biodegradable interconnector element comprises a plurality of first narrow strips spaced around a circumference of the first distal end portion and the second proximal end portion.

The multiple stent structure, further comprising a third stent segment having a third proximal end portion and a third distal end portion, wherein a second biodegradable interconnector element attaches between the second distal end portion and the third proximal end portion.

The multiple stent structure, wherein the first biodegradable interconnector element maintains the first and the second stent segments at a first spaced apart gap and the second biodegradable interconnector element maintains the second and the third stent segments at the first spaced apart gap, the first spaced apart gap ranging from about .15 mm to about 2 mm.

I.The multiple stent structure, wherein the second biodegradable interconnector element comprises a plurality of second narrow strips spaced around a circumference of the second distal end portion and the third proximal end portion.

The multiple stent structure, further comprising a second spacer element affixed to the outer surface of the inner catheter, the second spacer element having a second depth sufficient to prevent contact of the second and third stent segments during deployment when the outer sheath is positioned proximal relative to the inner catheter.

The multiple stent structure, wherein the second spacer element comprises a plurality of second segmented portions, each of the plurality of second segmented portions extending only partially around a circumference of the second distal end portion and the third proximal end portion.

The multiple stent structure, wherein the first and the second biodegradable interconnectors are positioned between the first and the second segmented portions.

The multiple stent structure, wherein the second biodegradable interconnector element prevents contact of the second distal end portion and the third proximal end portion within a body lumen.

The multiple stent structure, wherein the first biodegradable interconnector and the second biodegradable interconnector comprise a first longitudinal biodegradable strip, the first longitudinal biodegradable strip being continuous and extending from a proximal end of the generally tubular structure to a distal end of the generally tubular structure.

The multiple stent structure, further comprising a second longitudinal biodegradable strip and a third longitudinal biodegradable strip, the second and third longitudinal biodegradable strips being continuous and extending from the proximal end of the generally tubular structure to the distal end of the generally tubular structure, the second longitudinal biodegradable strip oriented about 120 degrees from the first longitudinal biodegradable strip, the third longitudinal biodegradable strip oriented between the first and second longitudinal biodegradable strips.

The multiple stent structure, wherein the first biodegradable interconnector comprises a biodegradable polymer.

The multiple stent structure, wherein said biodegradable polymer is a drug eluting polymeric carrier that comprises at least one bioactive.

The multiple stent structure, wherein said bioactive is selected from the group consisting of antiproliferative and anti-inflammatory agents.

A multiple stent delivery structure for deployment of more than one stent segment, the structure comprising:
a plurality of separate, self-expandable stent segments configured in linear order and disposed over an inner catheter, a first stent segment of the plurality of self-expandable stent segments having a first proximal end portion and a first distal end portion, a second stent segment of the plurality of self-expandable stent segments having a second proximal end portion and a second distal end portion,
the second proximal end portion being adjacent to the first distal end portion, and a first spacer element affixed to an outer surface of the inner catheter, the first spacer element situated between the first and second stent segments, the first spacer element having a first depth sufficient to prevent contact of the first;
and second stent segments during deployment of the first and second stent segments at a treatment site when the outer sheath is positioned proximal relative to the inner catheter.

The multiple stent delivery structure, further comprising a third stent segment having a third proximal end portion and a third distal end portion, the third proximal end portion being adjacent to the second distal end portion, a second spacer element affixed to the outer surface of the inner catheter, the second spacer element situated between the second and third stent segments, wherein the second spacer element has a second depth sufficient to prevent contact of the second and third stent segments during deployment of the first and the second and the third stent segments at the treatment site when the outer sheath is positioned proximal relative to the inner catheter

The multiple stent delivery structure, wherein the first spacer element is a molded structure affixed to the outer surface of the inner catheter, the molded structure completely extending around a circumference of the first distal end portion and the second proximal portion.

The multiple stent delivery structure, wherein the first spacer element is a washer or ring affixed to the outer surface of the inner catheter, the washer or ring completely extending around a circumference of the first distal end portion and the second proximal portion.

The multiple stent delivery structure, wherein the first spacer element comprises a plurality of first segmented portions, each of the plurality of first segmented portions extending only partially around a circumference of the first distal end portion and the second proximal end portion.

An intraluminal stent, comprising:
a first structural end, a second structural end, and a support structure spanning an entire length between the first structural end and the second structural end, the support structure comprising a plurality of interconnecting members and a plurality of open areas between the interconnecting members, the support structure thereby being expandable from a collapsed configuration to an expanded configuration, the support structure being non-biodegradable; and a biodegradable connector comprising a first end and a second end, the first end and the second end being connected to the interconnecting members of the support structure;
wherein the biodegradable connector extends across at least one of the open areas without crossing over the interconnecting members and the biodegradable connector extends along less than the entire length of the support structure. An intraluminal stent wherein the biodegradable connector comprises a width along a circumferential direction of the support structure of 0.25 mm (.010 inch) or less.

An intraluminal stent wherein the biodegradable connector comprises a suture.

An intraluminal stent wherein the biodegradable connector is aligned with a first interconnecting member and a second interconnecting member, the first interconnecting member and the second interconnecting member extending generally longitudinally, the first end of the biodegradable connector being attached to a second structural end of the first interconnecting member and the second end of the biodegradable connector being attached to a first structural end of the second interconnecting member.

An intraluminal stent wherein the first end of the biodegradable connector is tied around the second structural end of the first interconnecting member, a first angular interconnecting member extending obtusely from the second structural end of the first interconnecting member, and the second end of the biodegradable connector is tied within a bend formed between the first structural end of the second interconnecting member and a second angular interconnecting member extending acutely from the first structural end of the second interconnecting member.

An intraluminal stent wherein the biodegradable connector is made from PLA.

An intraluminal stent wherein the biodegradable connector comprises a width along a circumferential direction of the support structure of 0.25mm (.010 inch) or less, the biodegradable connector comprises a suture, and the biodegradable connector is made from PLA. An intraluminal stent wherein the biodegradable connector is aligned with a first interconnecting member and a second interconnecting member, the first interconnecting member and the second interconnecting member extending generally longitudinally, the first end of the biodegradable connector being attached to a second structural end of the first interconnecting member and the second end of the biodegradable connector being attached to a first structural end of the second interconnecting member, and the first end of the biodegradable connector is tied around the second structural end of the first interconnecting member, a first angular interconnecting member extending obtusely from the second structural end of the first interconnecting member, and the second end of the biodegradable connector is tied within a bend formed between the first structural end of the second interconnecting member and a second angular interconnecting member extending acutely from the first structural end of the second interconnecting member.

An intraluminal stent, comprising: a first structural end, a second structural end, and a support structure spanning an entire length between the first structural end and the second structural end, the support structure comprising a plurality of undulating rings, the undulating rings being connected to adjacent undulating rings with interconnecting members, the interconnecting members extending generally longitudinally between adjacent undulating rings and the interconnecting members being made from a same non-biodegradable material as the undulating rings; and
a biodegradable connector extending generally longitudinally between a first undulating ring and an adjacent second undulating ring, the biodegradable connector being connected to the first undulating ring and the second undulating ring, wherein the biodegradable connector is disposed between two adjacent interconnecting rings.

An intraluminal stent wherein the biodegradable connector comprises a first end and a second end, the first end being connected to the first undulating ring and the second end being connected to the second undulating ring, the biodegradable connector extending along less than the entire length of the support structure, and the biodegradable connector is circumferentially offset from all proximally adjacent interconnecting members, an open area being disposed proximally adjacent the biodegradable connector between the first undulating ring and a proximally disposed undulating ring, and the biodegradable connector is circumferentially offset from all distally adjacent interconnecting members, an open area being disposed distally adjacent the biodegradable connector between the second undulating ring and a distally disposed undulating ring.

An intraluminal stent wherein the biodegradable connector comprises a first end and a second end, the first end being connected to the first undulating ring and the second end being connected to the second undulating ring, the biodegradable connector extending along less than the entire length of the support structure, and the biodegradable connector is aligned with a proximally adjacent interconnecting member, the proximally adjacent interconnecting member being connected to the first undulating ring and a proximally disposed undulating ring, and the biodegradable connector is aligned with a distally adjacent interconnecting member, the distally adjacent interconnecting member being connected to the second undulating ring and a distally disposed undulating ring. An intraluminal stent wherein the biodegradable connector extends substantially across the entire length of the support structure.

An intraluminal stent wherein the biodegradable connector comprises a width along a circumferential direction of the support structure of 0.25 mm (.010 inch) or less.

An intraluminal stent, comprising:
a support structure comprising a first ring and an adjacent second ring, the first ring and the second ring generally defining a circumference of the support structure and
being longitudinally spaced apart;
an interconnecting member connected at one end to the first ring and connected at another end to the second ring, the interconnecting member being made from a non-biodegradable material; and
a biodegradable connector connected at one end to the first ring and connected at another end to the second ring, the biodegradable connector extending along less than an entire length of the support structure and being circumferentially spaced away from the interconnecting member.

An intraluminal stent wherein the biodegradable connector comprises a width along a circumferential direction of the support structure of 0.25 mm (.010 inch) or less. An intraluminal stent wherein the biodegradable connector comprises a suture.

An intraluminal stent wherein the interconnecting member and the biodegradable connector are longitudinally aligned with each other.

An intraluminal stent wherein the biodegradable connector is circumferentially offset from all proximally adjacent interconnecting members, an open area being disposed proximally adjacent the biodegradable connector between the first ring and a proximally disposed ring, and the biodegradable connector is circumferentially offset from all distally adjacent interconnecting members, an open area being disposed distally adjacent the biodegradable connector between the second ring and a distally disposed ring.

An intraluminal stent wherein the biodegradable connector is aligned with a proximally adjacent interconnecting member, the proximally adjacent interconnecting member being connected to the first ring and a proximally disposed ring, and the biodegradable connector is aligned with a distally adjacent interconnecting member, the distally adjacent interconnecting member being connected to the second ring and a distally disposed ring.

An intraluminal stent wherein the interconnecting member and the biodegradable connector are longitudinally aligned with each other.

An intraluminal stent, comprising:
a first supporting end, a second supporting end, and a plurality of struts extending between the first supporting end and the second supporting end to define a generally cylindrical body, the cylindrical body having a lumen and a longitudinal length, the struts being self-expandable from a collapsed configuration to an expanded configuration;
a stabilizing element comprising a first end, a second end, and a fixed length therebetween, the first end connected to a first strut and the second end connected to a second strut; and
wherein the stabilizing element spans between the first strut and the second strut without obstructing the lumen to supplement the stiffness of the stent.

The intraluminal stent, wherein the first end is connected to the first supporting end and the second end is connected to the second supporting end.

The intraluminal stent, wherein the stabilizing element comprises a suture.

The intraluminal stent, wherein the suture extends along the struts.

The intraluminal stent, wherein the stabilizing element is biodegradable.

The intraluminal stent, wherein the first end of the stabilizing element is tied around one of the plurality of struts at the first supporting end, and further wherein the second end of the stabilizing element is tied around one of the plurality of struts at the second supporting end.

The intraluminal stent, wherein the first end of the stabilizing element is bonded to one of the plurality of the struts at the first supporting end and the second end of the stabilizing element is bonded to one of the plurality of the struts at the second supporting end.

The intraluminal stent, wherein the stabilizing element extends about the cylindrical body and along the struts in a helical manner.

An intraluminal stent, comprising:
a first supporting end, a second supporting end, and a plurality of struts extending between the first supporting end and the second supporting end to define a generally cylindrical body having a lumen and a longitudinal length, the struts being self-expandable from a collapsed configuration to an expanded configuration;
one or more stabilizing polymeric strips comprising a first end, a second end, and a fixed length therebetween, the first end affixed to a first strut and the second end affixed to a second strut end; and
wherein the one or more stabilizing polymeric strips extends between the first strut and the second strut to supplement the stiffness of the stent.

The intraluminal stent, wherein the one or more stabilizing polymeric strips extends linearly along the longitudinal length of the cylindrical body to connect adjacent struts.

The intraluminal stent, wherein the one or more polymeric strips continuously extends along all of the longitudinal length of the cylindrical body.

The intraluminal stent, wherein the one or more polymeric strips comprises frangible zones.

The intraluminal stent, further comprising a plurality of strips, wherein a first portion of the strips extend substantially linearly along the longitudinal length of the cylindrical body and a second portion of the strips extend in a helical fashion about the cylindrical body to provide torsional stiffness.

The intraluminal stent, each of the one or more strips are circumferentially spaced apart from each other.

The intraluminal stent, wherein the strips are formed from a polyetherurethaneurea blended with a surface modifying siloxane-based additive.

An intraluminal stent, comprising:
a first supporting end, a second supporting end, and a plurality of struts extending between the first supporting end and the second supporting end to define a generally cylindrical body having a lumen and a longitudinal length, the struts being self-expandable from a collapsed configuration to an expanded configuration;
one or more stabilizing sutures comprising a first end, a second end and a fixed length therebetween, the first end connected to a first strut and the second end connected to a second strut; and wherein the one or more stabilizing sutures extends between the first strut and the second strut to supplement the stiffness of the stent.

The intraluminal stent, wherein the one or more stabilizing sutures extends substantially linearly along the longitudinal length of the cylindrical body.

The intraluminal stent, wherein the suture is formed from a biodegradable material.

The intraluminal stent, wherein the biodegradable material comprises a copolymer of glycolide and L-lactide.

The intraluminal stent, wherein the suture comprises a diameter of about .07 mm.

The intraluminal stent, further comprising a plurality of sutures, wherein a first portion of the sutures extends substantially linearly along the longitudinal length of the cylindrical body and a second portion of the sutures extends in a helical fashion about the cylindrical body to provide torsional stiffness.

The intraluminal stent, further comprising a plurality of sutures that extends substantially linearly along the longitudinal length of the cylindrical body, each of the plurality of sutures spaced apart along a circumference of the cylindrical body of the stent.

A method of manufacturing a stent with stabilizing elements, comprising the steps of: inserting an expandable stent into a transfer tube, the transfer tube comprising one or more slots; expanding the stent partially within the slotted transfer tube; spraying a semi-rigid or rigid polymeric material through the one or more slots onto an outer surface of a plurality of struts of the stent; and curing the sprayed outer surface

The method of manufacture, wherein the curing is achieved at a predetermined temperature and pressure for a predetermined time.

The method of manufacture, wherein the polymeric material comprises polyetherurethaneurea blended with a surface modifying siloxane-based additive.

The method of manufacture, further comprising the step of withdrawing the stent from the slotted transfer tube.

An intraluminal stent, comprising: a first supporting end, a second supporting end, and a plurality of struts extending between the first supporting end and the second supporting end to define a generally cylindrical body having a lumen and a longitudinal length, the struts being self-expandable from a collapsed configuration to an expanded configuration; one or more stabilizing polymeric strips comprising a first end, a second end, and a fixed length therebetween, the first end affixed to a first supporting end and the second end affixed to a second supporting end, the one or more stabilizing polymeric strips comprising frangible zones; and wherein the one or more stabilizing polymeric strips extends between the first strut and the second strut to supplement the stiffness of the stent.

The intraluminal stent, wherein the frangible zone comprises geometric discontinuities.

The intraluminal stent, wherein the frangible zone has fracture planes.

The intraluminal stent, wherein the fracture planes controllably break upon radial expansion of the stent.

### Brief Description of the Drawing

The invention may be more fully understood by reading the following description in conjunction with the drawings, in which:
Figure 1 is a partial top view of the delivery device holding three interconnected stent segments, the stent segments being shown in side view;
Figure 2 is a partial top view of three stent segments held together by multiple biodegradable longitudinal strips, the stent segments shown in side view;
Figure 3A is a side view of three stent segments held together by a sleeve;
Figure 3B is an end view of the sleeve of Figure 3A;
Figure 4 is a partial top view of a multiple stent delivery structure that is capable of delivering and deploying several self-expandable stent segments at various treatment sites;
Figure 5 is a side view of two segmented stents connected by a non-biodegradable sleeve having selected round openings therethrough.
Figure 6 is a plan view of a stent;
Figure 7 is a plan view of a portion of a stent, showing biodegradable connectors;
Figure 8 is a plan view of a portion of another stent, showing biodegradable connectors;
Figure 9 is a plan view of a portion of another stent, showing biodegradable connectors;
Figure 10 is a plan view of a portion of another stent, showing a biodegradable suture;
Figure 11 is a side view of a stent with a stabilizing element;
Figure 12 is blown-up view of Figure 11 showing a first end of the stabilizing element connected to an eyelet of the stent;
Figure 13 is a side view of the stent of Figure 11 loaded into a delivery system;
Figure 14 is a side view of the stent of Figure 13 deployed;
Figure 15 is a perspective view of the stent of Figure 11 having various stabilizing strips and stabilizing segments;
Figure 16 is a perspective view of the stent of Figure 11 having polymeric segments; and
Figure 17 is a top view of a stabilizing strip with three frangible zones in which each frangible zone has geometric discontinuities.

### Detailed Description

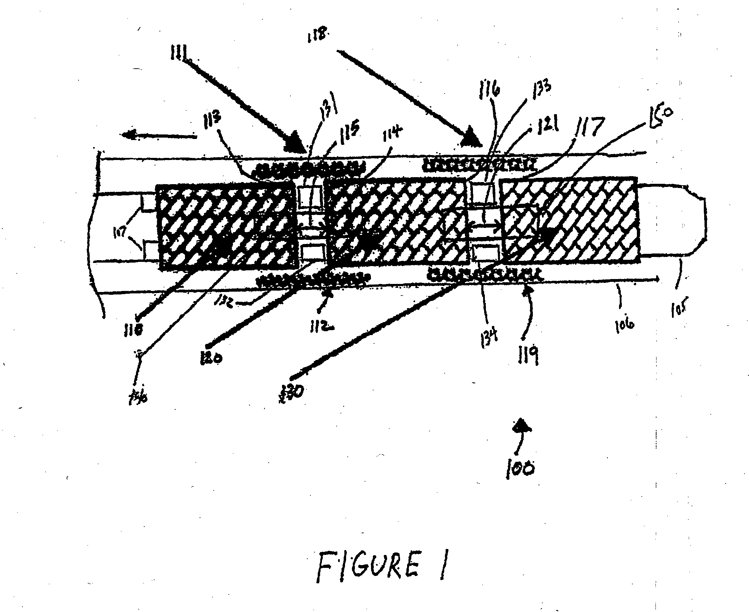

An exemplary segmented stent structure 100 is shown in Figure 1. Figure 1 is a partial top view showing three expandable stents 110, 120, 130 configured in a linear order and mounted onto an inner catheter 105. Stent segment 110 is the most proximal situated stent segment and stent segment 130 is the most distal situated stent segment. Each expandable stent segment 110, 120, 130 is designed to radially expand from a compressed state to an expanded state. Figure 1 shows the stents 110, 120, 130 in a compressed state with an outer sheath 106 disposed over and constraining the stents 110, 120, 130. The expandable stent segments 110, 120, 130 may be self-expanding. The segments 110, 120, 130 may be formed from a suitable metallic alloy such as stainless steel, NITINOL or any other suitable biocompatible material.

Generally speaking, the linear order of stent segments 110, 120, and 130 are held together by biodegradable interconnectors. The term "biodegradable" as used herein is intended to encompass any type of material that breaks down and/or is absorbed and loses its structural rigidity over time. The term "biostable" as used herein refers to any material which does not break down and/or absorb and lose its structural rigidity over time. Each of the biodegradable interconnectors connect the distal end portion of one stent to the proximal end portion of an immediately adjacent stent. The biodegradable interconnectors enable the segmented stent structure 100 to possess relatively greater longitudinal and torsional flexibility than is available with current stent designs that have a greater length. After implantation of the expandable stent segments 110, 120, 130 at a target site, the biodegradable interconnectors degrade over a predetermined time leaving behind only the unconnected stent segments 110, 120, 130. Longitudinal and torsional flexibility along the stented region may be improved by having a series of unconnected stent segments rather than a single stent that is longer than that of stent segments 110, 120, 130.

The details of Figure 1 will now be discussed. Biodegradable interconnector 111 is shown extending along a first side of stent segments 110 and 120 and biodegradable interconnector 112 is shown extending along a second side of stent segments 110 and 120. A third biodegradable interconnector 140 is shown extending along the top of stent segments 110 and 120. Biodegradable interconnectors 111, 112, and 140 connect the distal end portion 113 of stent segment 110 with the proximal end portion 114 of stent segment 120. The interconnectors 111, 112, 140 maintain a predetermined gap 115 between stent segment 1110 and 120. The predetermined gap 115 preferably has a range between about .05 mm to about 4 mm, and more preferably between .15 mm to about 2 mm. Because the stent segments 110 and 1 20 are deployed at a target site with a predetermined gap 115 between them, the end portions of the stent segments 110, 120 preferably do not overlap and hit each other to prevent significant twisting, kinking, rubbing and cutting action, all of which can potentially cause breakage of the stent segments 110 and 120.

Still referring to Figure 1, biodegradable interconnector 118 is shown extending along a first side of stent segments 120 and 130 and biodegradable interconnector 119 is shown extending along a second side of stent segments 120 and 130. A third biodegradable interconnector 1 50 is shown extending along the top of stent segments 120 and 130. Biodegradable interconnectors 118, 119, and 150 connect the distal end portion 116 of stent segment 120 with the proximal end portion 117 of stent segment 130. The interconnectors 118, 119, 150 maintain a predetermined gap 121 between stent segments 120 and 130.

Although Figure 1 shows predetermined gaps 115 and 121 to be the same distance, the gaps 115, 121 may be different depending on the type of structure the segmented stent structure 100 is implanted within. For example, the stricture that segmented stent structure 100 is implanted within may have a geometry requiring segmented stent 120 to be in closer proximity to segmented stent 110 than with segmented stent 130. Accordingly, the stent structure 100 may be designed such that gap 11 5 is a smaller distance than gap 121.

Figure 1 indicates that interconnectors 111, 112, 140 and 118, 119, 150 do not completely circumscribe their respective stent segments 110, 120, and 130. Rather, they partially extend circumferentially around the segments 110, 120, and 130. Preferably, the interconnectors 111, 112, 140 and 118, 119, 150 are longitudinal narrow strips that are aligned along the longitudinal direction of the stent segments 110, 120, 130. The strips have a width that is sufficiently narrow to permit the stent segments 110, 120, and 130 to be delivered through tortuous body lumens. Additionally, the strips are sufficiently narrow to not impede radial expansion of the stent segments 110, 120, and 130 from their compressed state. The strips are adequately flexible such that radial expansion may not cause them to break off from the surface of stent segments 110, 120, and 130.

The interconnectors 111, 112, 140, and 118, 119, 150 may overlap the end portions of their respective stent segments 110, 120, 130 so as to create sufficient contact between the interconnectors 111, 112, 140, 118, 119, 150 and the end portions 113, 114, 116, and 117. This may enable the stent segments 110, 120, 130 to be held together as a unitary segmented stent structure 100 during delivery, deployment, and also for a finite time during post-deployment. The degree of overlap preferably ranges between about 1 mm to about 5 mm, and more preferably between about 2 mm to about 4 mm.

Any number of interconnectors between adjacent stent segments are contemplated. The actual number of interconnectors used to connect adjacent stent segments will be dependent on many factors including the stricture that the segmented stent structure 100 is to be implanted within, the length of the stricture, and the desired flexibility and stiffness of the segmented stent structure 100. Each end portion of each of the stent segments may have an unequal number of interconnectors in order for the stent structure 100 to achieve a desired flexibility and stiffness.

The thickness of the interconnectors 111, 112, 140, 118, 119, 150 may vary depending on numerous factors, including the thickness of the struts of the stent segments 110, 120, 130, the region where the structure 100 is to be implanted, and the degree of flexibility and stiffness desired from the structure 100. Preferably, the interconnectors 111, 112, 140, 118, 119, 150 within the gaps 115 and 121 are thicker than the struts of the stent segments 110, 120, 130 so that the struts can be embedded within the interconnectors 1 1 1, 1 12, 140. Similarly, interconnectors 118, 119, 150 are preferably thicker than the struts at end portions 116 and 117.

Still referring to Figure 1, spacer elements 131 and 132 are situated between stent segment 110 and 120. Spacer elements 133, 134 are situated between stent segments 120 and 130. The spacer elements 131, 132, 133, 134 allow the individual stent segments 110, 120, 130 of the multiple stent segment structure 100 to be deployed using a standard inner catheter 105 and outer sheath 106 as is known in the art. The spacer elements 131, 132, 133, 134 are affixed to an outer surface of the inner catheter 105. The depth of spacer elements 131, 132 prevent the second stent segment 120 from being pulled proximally back with the outer sheath 106 during proximal movement of outer sheath 106 relative to the inner catheter 105, as indicated by the arrow. Similarly, the depth of spacer elements 133, 134 prevent the third stent segment 1 30 from being pulled proximally back with the outer sheath 106 during proximal movement of outer sheath 106 relative to the inner catheter 105. Stops 107 prevent the proximal-most stent segment 110 from being pulled proximally with proximal movement of the outer sheath 106 relative to the inner catheter 105. The spacer elements 131, 132, 133, 134 may have ring-like structures which are circumferentially positioned around the inner catheter 105. Because there is only enough depth for either the biodegradable interconnectors 111, 112, 140 and 118, 119, 150 or the spacer elements 131-134, each of the elements 131-134 and each of the interconnectors 111, 112, 118, 140, 119, 150 occupy their own circumferential position about inner catheter 105 such that neither the spacer elements 131, 132, 133, 134 nor the interconnectors 111, 112, 140, 118, 119, 150 interfere with each other. The interconnectors 111, 112, 140, 118, 119, 150 and the spacer elements 131, 32, 133, 134 may be configured in an alternating or staggered arrangement relative to each other.

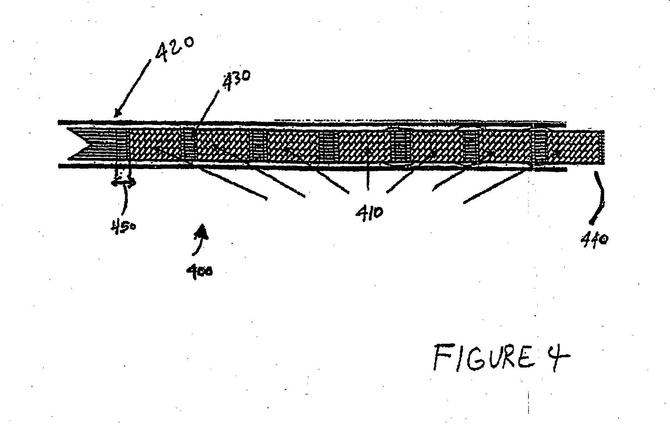

Various types of spacer elements 131, 132, 133, 134 are contemplated. For example, the spacer elements 131, 132, 133, 134 may be segmented portions, such as structural ribs, that can be affixed to the outer wall of the catheter 105 by any method known to one of ordinary skill in the art, including adhesion. Alternatively, the spacer elements 430, shown in Figure 4, may be molded onto the catheter 440 as segmented rings or knobs.

Referring back to Figure 1, as an alternative to having spacer elements 131-134 to permit deployment of the stent segments 110, 120, 130 with a standard catheter 105 and outer sheath 106, a portion of the surfaces of each of the stent segments 110, 120, 130 may be sufficiently embedded into the plastic of the catheter 105 such that the stent segments 110, 120, 130 do not move proximally with proximal movement of the outer sheath 106 during deployment. This technique is fully described in U.S. Patent Published Application No. 2006-0206187. According to this patent application, the inner catheter 105 affixes to the inner surface of the stent segments 110, 120, 130. One method of affixing the inner catheter 105 to the stent segments 110, 120, 130 is by blow molding the inner catheter 105 under suitable heat and pressure blow molding parameters. The inner catheter 105 may be formed from a variety of suitable polymeric materials, including polyethylene terephthalate (PET). The blow molded inner catheter 105 maintains the stent segments 110, 120, 130 spaced apart during delivery and deployment of the stent segments 110, 120, 130.

The resultant stent structure is then inserted into a delivery sheath 106. As the structure arrives at the deployment site, the delivery sheath 106 is pulled proximally back to expose stent segments 110, 120, and 130. Because the outer surface of the inner catheter 105 is molded to the stent segments 110, 120, 130, the catheter 105 temporarily restrains a longitudinal movement of the stent segments 110, 120, 130. When the expansion force of the stent segments 110, 120, 130 exceeds the restraining force of the catheter 105, the stent segments 110, 120, 130 start to expand.

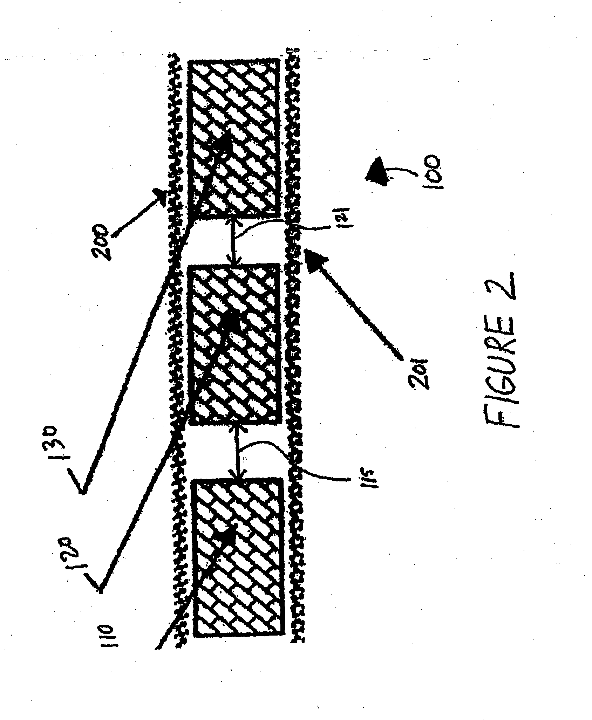

Figure 2 shows an alternative segmented stent structure 100 in which the interconnectors 111, 112, 118, 119 are replaced by two continuous biodegradable strips 200 and 201. Biodegradable strips 200 and 201 extend from the proximal end portion of stent segment 110 to the distal end portion of stent segment 130. Biodegradable strip 200 extends the entire length along a first circumferential side and biodegradable strip 201 extends the entire length along a second circumferential side of stent segments 110, 120, and 130. In this embodiment, biodegradable strip 201 is oriented about 180 degrees relative to biodegradable strip 200. The strips 200 and 201 may be oriented at other angular configurations. Although not shown in Figure 2 for clarity, an additional strip may be oriented between strips 200 and 201 and also extend the entire length of the segmented stent structure 100. Preferably, the angles between pairs of the three strips will be about 120 degrees.

Referring back to Figure 1, the interconnectors 111, 112, 118, 140, 119 and 1 50 can comprise a biodegradable material that can be degraded and/or absorbed by the body over time to advantageously provide a flexible stented region that comprises a series of relatively shorter length unconnected stent segments than typical stent designs. Desirably, any polymer that is used adequately adheres to the surface of the stent segments 110, 120, 130 and deforms readily after it is adhered to the device. The molecular weight of the polymer(s) should be high enough to provide sufficient toughness so that the polymers will not be rubbed off during sterilization, handling, or deployment of the stent segments 110, 120, 130 and will not crack when the stent segments 110, 120, 130 are expanded. Exemplary polymer systems that may be used include polymers that minimize irritation when the segmented stents 110, 120, 130 are implanted.

A biodegradable polymer may be preferred in certain embodiments because, unlike a biostable polymer, it will not be present long after implantation to cause any adverse, chronic local response. The properties of any mixture of polymers depend primarily on thermodynamic miscibility. If the polymers are immiscible, the properties will depend not only on the properties of each component, but also on the morphology and adhesion between the phases.

Desirably, the biodegradable polymer comprises a polylactic acid (PLA), which may be a mixture of enantiomers typically referred to as poly-D, L-lactic acid. PLA is one of the poly-α-hydroxy acids, which may be polymerized from a lactic acid dimer. This polymer has two enantiomeric forms, poly(L-lactic acid) (PLLA) and poly(D-lactic acid) (PDLA), which differ from each other in their rate of biodegradation. PLLA is semicrystalline, whereas PDLA is amorphous, which may be desirable for applications such as drug delivery where it is important to have a homogeneous dispersion of an active species. PLA has excellent biocompatibility and slow degradation, is generally more hydrophobic than polyglycolic acids (PGA). The polymer used may also desirably comprise polyglycolic acids (PGA). Polyglycolic acid is a simple aliphatic polyester that has a semi-crystalline structure, fully degrades in 3 months, and undergoes complete strength loss by 1 month. Compared with PLA, PGA is a stronger acid and is more hydrophilic, and, thus, more susceptible to hydrolysis.

Other desirable biodegradable polymers for use include, but are not limited to, polylactic glycolic acids (PLGA) and other copolymers of PLA and PGA. The properties of the copolymers can be controlled by varying the ratio of PLA to PGA. For example, copolymers with high PLA to PGA ratios generally degrade slower than those with high PGA to PLA ratios.

Still other desirable polymers for use include poly(ethylene glycol) (PEG), polyanhydrides, polyorthoesters, fullerene, polytetrafluoroethylene, poly(styrene-b-isobutylene-b-styrene), polyethylene-co-vinylacetate, poly-N-butylmethacrylate, amino acid-based polymers (such as poly(ester) amide), SiC, TiNO, Parylene C, heparin, porphorylcholine.

A number of biodegradable homopolymers, copolymers, or blends of biodegradable polymers are known in the medical arts. These include, but are not limited to: polyethylene oxide (PEO), polydioxanone (PDS), polypropylene fumarate, poly(ethyl glutamate-co-glutamic acid), poly(tert-butyloxy-carbonylmethyl glutamate), polycaprolactones (PCL), polyhydroxybutyrates (PHBT), polyvalerolactones, polyhydroxyvalerates, poly(D,L-lactide-cocaprolactone) (PLA/PCL), polycaprolactone-glycolides (PGA/PCL), poly(phosphate ester), and poly(hydroxy butyrate), polydepsipeptides, maleic anhydride copolymers, polyphosphazenes, polyiminocarbonates, polyhydroxymethacrylates, polytrimethylcarbonates, cyanoacrylate, polycyanoacrylates, hydroxypropylmethylcellulose, polysaccharides (such as hyaluronic acid, chitosan and regenerate cellulose), fibrin, casein, and proteins (such as gelatin and collagen), poly-e-decalactones, polylactonic acid, polyhydroxybutanoic acid, poly(1,4-dioxane-2,3-diones), poly(1,3-dioxane-2-ones), poly-p-dioxanones, polyb-maleic acid, polycaprolactonebutylacrylates, multiblock polymers, polyether ester multiblock polymers, poly(DTE-co-DT-carbonate), poly(N-vinyl)-pyrrolidone, polyvinylalcohols, polyesteramides, glycolated polyesters, polyphosphoesters, poly[p-carboxyphenoxy)propane], polyhydroxypentanoic acid, polyethyleneoxidepropyleneoxide, polyurethanes, polyether esters such as polyethyleneoxide, polyalkeneoxalates, lipides, carrageenanes, polyamino acids, synthetic polyamino acids, zein, polyhydroxyalkanoates, pectic acid, actinic acid, carboxymethylsulphate, albumin, hyaluronic acid, heparan sulphate, heparin, chondroitinesulphate, dextran, b-cyclodextrines, gummi arabicum, guar, collagenN-hydroxysuccinimide, lipides, phospholipides, resilin, and modifications, copolymers, and/or mixtures of any of the carriers identified herein.

Other suitable biodegradable polymers that may be used include, but are not limited to: aliphatic polyesters (including homopolymers and copolymers of lactide), poly(lactide-co-glycolide), poly(hydroxybutyrate-co-valerate), poly(hydroxybutyrate-co-hydroxyvalerate) (PHBV), polyoxaster and polyoxaesters containing amido groups, polyamidoester, poly(glycolic acid-co-trimethylene carbonate), poly(trimethylene carbonate), and biomolecules and blends thereof such as fibrinogen, starch, elastin, fatty acids (and esters thereof), glucosoglycans, and modifications, copolymers, and/or mixtures or combinations of any of the carriers identified herein.

The polymeric materials of biodegradable interconnectors 111, 112, 140, 118, 119, 150 preferably remain attached to stent segments 110, 120, 130 for at least about a month after implantation within a vessel in order to allow endothelial tissue to grow and fixate the stent segments 110, 120, 130 within the vessels.

Countervailing design attributes of rigidity and flexibility are desirable for the segmented stent structure 100. On the one hand, it is desirable for the segmented stent structure 100 to be sufficiently rigid such that the stent segments 110, 120, 130 maintain separation from each within the implanted vessel. Poor rigidity of the stent segments 110, 120, 130 can cause the ends of the stent segments 110, 120, 130 to contact each other, thereby potentially kinking and rubbing so as to ultimately damage the struts. On the other hand, it is desirable for the segmented stent structure 100 to be sufficiently flexible so that the individual stent segments 110, 120, 130 are capable of adapting to a moving artery, such as the SFA. Numerous design variables affect its rigidity and flexibility, including the strut thickness, the length of each of the stent segments 110, 120, 130, the biodegradable material used for the interconnectors 111, 112, 140, 118, 119, 150, the thickness of the biodegradable interconnectors, 112, 140, 118, 119, 150, and the vessel that the segmented stent structure 100 is to be implanted within. One of ordinary skill in the art would be able to select the desired design variables to achieve the desired flexibility and stiffness.

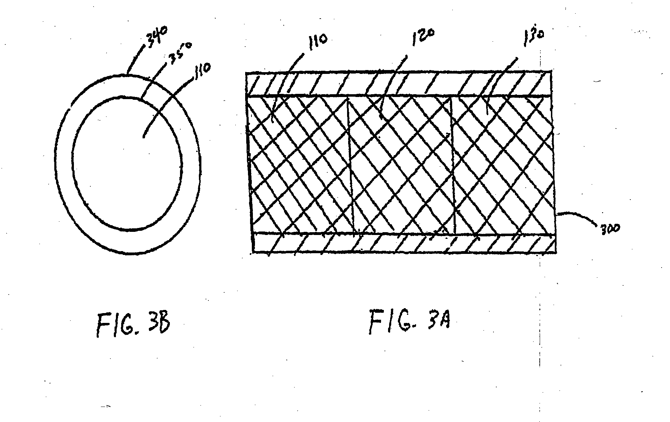

As an alternative to the continuous biodegradable strips 200 and 201 shown in Figure 2 and the biodegradable interconnectors 111, 112, 140, 118, 119, 150 shown in Figure 1, a biodegradable sleeve 300 may also be used, as shown in Figures 3A and 3B. The sleeve 300 is generally a cylindrical structure that extends from a proximal end of stent segment 110 to a distal end of segmented stent 130. The sleeve 300 has an inner wall 350 and an outer wall 340. Figure 3B is an end view of the biodegradable sleeve 300.

The continuous material of the sleeve 300 provides it with sufficient strength to hold all of the stent segments 110, 120, 130 together as a unitary structure. Upon deployment, the sleeve 300 may be biodegradable over time, thereby eventually releasing stent segments 110, 120, and 130 and allowing them to move relative to one another. The degradation of the sleeve 300 allows the stent segments 110, 120, and 130 to independently move and adapt to the various body lumens in which they are implanted. The sleeve 300 may be formed from any of the biodegradable materials described above.

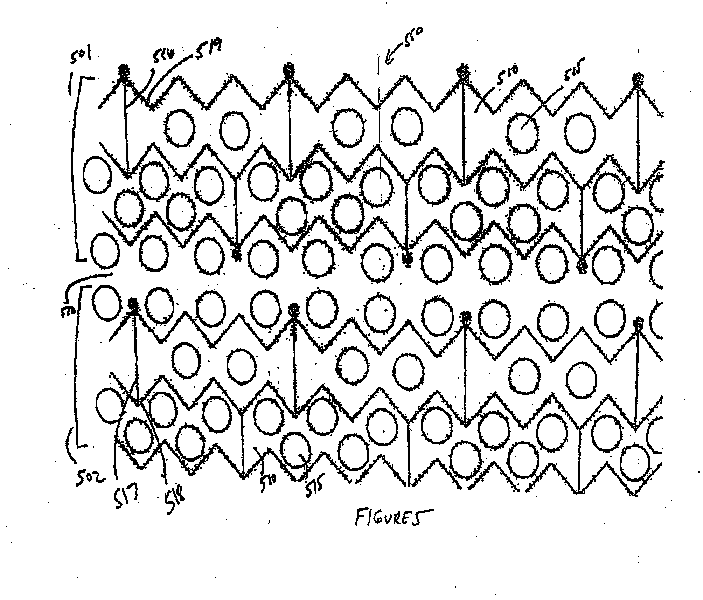

The sleeve 300 may also be formed from a non-biodegradable material. The sleeve 300 could contain select openings to allow contents to flow through the stents, thereby preventing occlusion of side lumens so that blood can flow and carry nutrients to the healthy tissue surrounding the side lumens. Additionally, the openings would allow endothelial cells to line the inner surfaces of the stent segments 110, 120, 130. An example of such a biostable sleeve with openings is shown in Figure 5. Figure 5 shows a segmented stent structure 550 composed of stent segments 501, 502 and a non-biodegradable sleeve 510 encapsulating the segments 501, 502. The stent segments 501 and 502 are depicted side by side along a vertical axis. Stent segment 501 is composed of two cells that contain struts 519 arranged in a zigzag pattern of rows with interconnectors 516 connecting adjacent rows of the struts 519. Similarly, stent segment 502 is composed of two cells that contain struts 518 arranged in a zigzag pattern of rows with interconnectors 517 connecting adjacent rows of the struts 518. There is no strut connecting stent segment 501 and 502. Rather, the non-biodegradable sleeve 510 provides the only medium by which the stent segments 501, 502 are in communication with each other. The sleeve 510 enables the stent segments 501, 502 to possess sufficient flexibility to adapt to the continuous bending encountered in vessels, such as the SFA, yet at the same time, provides enough rigidity for the segmented stent structure 550 to remain fixated within a target region of a vessel. Additionally, because the non-biodegradable sleeve 510 permanently remains over the segments 501 and 502, multiple openings 515 are created throughout the sleeve 510 to prevent occlusion of any side branch vessels. The openings 51 5 also allow endothelial tissue to migrate or grow from the vessel inner wall and grow through the openings 515 and onto the luminal surfaces of the struts 519 of stent segment 501 and the struts 518 of stent segment 502. Endothelial growth on the luminal surfaces of the struts 518, 519 and sleeve 510 may be favorable because it may prevent thrombosis formation and smooth muscle cell growth in the stent lumen.

The openings 515 in the sleeve 510 may constitute about 5% to about 80% of the circumferential surface area of the sleeve 510. The desired percentage of the total circumferential surface area that the openings constitute is dependent upon numerous design variables, including the stent strut pattern, and the length and diameter of the segmented stent structure 550. Figure 5 shows that the openings 515 constitute about 25% of the total circumferential surface area.

The openings 515 may be holes or pores. Holes are larger than the pores and may be mechanically formed by, for example, punching holes through the sleeve 510. Alternatively, a laser may be used to create the desired pattern of holes. Pores are significantly smaller than the holes and may be chemically formed. One example of chemical formation of the pores includes mixing salt with a polyurethane based polymer known as THORALON, which is a preferred biostable material that will be discussed in further detail below. THORAON is a polyetherurethaneurea blended with a surface modifying siloxane-based additive. The resultant salt-THORALON mixture may be sprayed onto the stent struts 518, 519. The salt is subsequently dissolved out of the THORALON to cause formation of the pores. The overall porosity of the sleeve 510 may be varied by altering the mass ratio of salt to THORALON. A suitable size of holes or pores will be dependent upon numerous factors, including the geometry and size of the strictures, the number of stent segments being used, the size and the number of healthy side lumens that would be occluded in the absence of the openings, and the extent of endothelialization required to cover the struts.

The openings 515 shown in Figure 5 do not intercept any of the struts 519 of stent segment 501 or any of the struts 518 of stent segment 502. Rather, the openings 515 are positioned so that they reside within adjacent rows of struts 519, 518. The openings may be generally circular shaped. Other variations of circular shaped are contemplated, such as elliptical shaped.

Any suitable biocompatible material may be used to fabricate the sleeve 510, including silicone, polyurethane, or combinations thereof. A preferred material for forming the sleeve would be elastomeric such that the material can readily compress during delivery and radially expand upon deployment. Preferably, a biocompatible polyetherurethaneurea blended with a surface modifying siloxane-based additive is used. One example of such a modified polyetherurethaneurea is THORLAON. THORALON is available from THORATEC in Pleasanton, CA. THORALON has been used in certain vascular applications and is characterized by thromboresistance, high tensile strength, low water absorption, low critical surface tension and good flex life. THORALON and methods of manufacturing this material are disclosed in U.S. Pat. Application Publication No. 2002/0065552 and U.S. Pat. Nos. 4,861,830 and 4,675,361, each of which is incorporated herein by reference in their entirety. According to these patents, THORALON is a polyurethane based polymer (referred to as BPS- 21 5) blended with a siloxane containing surface modifying additive (referred to as SMA-300). Base polymers containing urea linkages can also be used. The concentration of the surface modifying additive may be in the range of 0.5% to 5% by weight of the base polymer.

THORALON can be manipulated to provide either a porous or non-porous material. Formation of porous THORALON is described, for example, in U.S. Pat. No. 6,752,826 and 2003/0149471. The pores in the polymer may have an average pore diameter from about 1 micron to about, 400 microns. Preferably, the pore diameter averages from about 1 micron to about 100 microns, and more preferably averages from about 10 microns to about 100 microns.

A variety of other biocompatible polyurethanes/polycarbamates and urea linkages (hereinafter "-C(O)N or CON type polymers") may also be employed as the sleeve 300 material. Biocompatible CON type polymers modified with cationic, anionic and aliphatic side chains may also be used. See, for example, U.S. Pat. No. 5,017,664.

Other biocompatible CON type polymers include: segmented polyurethanes, such as BIOSPAN; polycarbonate urethanes, such as BIONATE; polyetherurethanes, such as ELASTHANE; (all available from POLYMER TECHNOLOGY GROUP, Berkeley, CA); siloxane-polyurethanes, such as ELAST-EON 2 and ELAST-EON 3 (AORTECH BIOMATERIALS, Victoria, Australia); polytetramethyleneoxide (PTMO) and polydimethylsiloxane (PDMS) polyether-based aromatic siloxane-polyurethanes, such as PURSIL-10, -20, and -40 TSPU; PTMO and PDMS polyether-based aliphatic siloxane-polyurethanes, such as PURSIL AL-5 and AL-10 TSPU; aliphatic, hydroxy-terminated polycarbonate and PDMS polycarbonate-based siloxane-polyurethanes, such as CARBOSIL-10, -20, and -40 TSPU (all available from POLYMER TECHNOLOGY GROUP). Examples of siloxane-polyurethanes are disclosed in U.S. Pat. Application Publication No. 2002/0187288.

In addition, any of these biocompatible CON type polymers may be end- capped with surface active end groups, such as, for example, polydimethylsiloxane, fluoropolymers, polyolefin, polyethylene oxide, or other suitable groups. See, for example the surface active end groups disclosed in U.S. Pat. No. 5,589,563. The biocompatible polyurethane, described herein, may be applied using any technique known in the art, including dipping, spraying, and electrospinning. In a preferred embodiment, the biocompatible polyurethane may be applied, as a solution.

Biostable polymers having a relatively low chronic tissue response may also be used. Such polymers may include, but are not limited to: polyurethanes, silicones, polyesters, poly(meth)acrylates, polyalkyl oxides, polyvinyl alcohols, polyethylene glycols, polyvinyl pyrrolidone, and hydrogels. Other polymers that may be dissolved and dried, cured or polymerized on the stent may also be used. Such polymers include, but are not limited to: polyolefins, polyisobutylene and ethylene-alphaolefin copolymers; acrylic polymers (including methacrylate) and copolymers, vinyl halide polymers and copolymers, such as polyvinyl chloride; polyvinyl ethers, such as polyvinyl methyl ether; polyvinylidene halides, such as polyvinylidene fluoride and polyvinylidene chloride; polyacrylonitrile, polyvinyl ketones; polyvinyl aromatics; copolymers of vinyl monomers with each other and olefins; polyamides; alkyd resins; polycarbonates; polyoxymethylenes; polyimides; polyethers; epoxy resins; polyurethanes; rayon; rayon-triacetate; cellulose; cellulose acetate; cellulose butyrate; cellulose acetate butyrate; cellophane; cellulose nitrate; cellulose propionate; cellulose ethers; and modifications, copolymers, and/or mixtures of any of the carriers identified herein. The polymers may contain or be coated with substances that promote endothelialization and/or retard thrombosis and/or the growth of smooth muscle cells.

The biodegradable. sleeve 300 discussed in Figure 3 and the nonbiodegradable sleeve 510 discussed in Figure 5 may be deployed using a conventional delivery catheter and outer sheath as is known to one of ordinary skill in the art. The materials that the biodegradable sleeve 300 and nonbiodegradable sleeve 510 may be formed from will resist being pulled back with the outer sheath 106 when the outer sheath 106 is proximally withdrawn during deployment relative to the inner catheter 105. This is possible because of the low coefficient of friction between the outer sheath 106 and the biodegradable sleeve 300 or the non- biodegradable sleeve 510. The low coefficient of friction between the biodegradable sleeve 300 or the non- biodegradable sleeve 510 and the outer sheath 106 may be achieved by applying a lubricious coating onto the outer surfaces of the biodegradable sleeve 300 or the non- biodegradable sleeve 510. For example, the lubricious coating may be a hydrophilic polymer. The hydrophilic polymer may be selected from the group comprising polyacrylate, copolymers comprising acrylic acid, polymethacrylate, polyacrylamide, poly(vinyl alcohol), poly(ethylene oxide), poly(ethylene imine), carboxymethylcellulose, methylcellulose, poly(acrylamide sulphonic acid), polyacrylonitrile, poly(vinyl pyrrolidone), agar, dextran, dextrin, carrageenan, xanthan, and guar. The hydrophilic polymers can also include ionizable groups such as acid groups, e.g., carboxylic, sulphonic or nitric groups. The hydrophilic polymers may be crosslinked through a suitable cross-binding compound. The cross-binder actually used depends on the polymer system: If the polymer system is polymerized as a free radical polymerization, a preferred cross-binder comprises 2 or 3 unsaturated double bonds.

Alternatively, the lubricious coating may be any biostable hydrogel as is known in the art. Alternatively, if expanded polytertrafluoroethylene (ePTFE) is used as the sleeve material, the stent segments may be deployed without an overlying lubricious coating. The lubricious coating may also promote cellular growth of endothelium into the stents or the gaps between them that fixes the stents in place in the artery. It may also contain or be coated with substances that promote endothelialization and/or retard thrombosis and/or the growth of smooth muscle cells.

As an alternative to a sleeve 510 with select openings as shown in Figure 5, the sleeve 510 may be configured as a spiral helix. In addition to providing openings for blood flow and endothelial cell growth along the surfaces of the stent segments, the spiral helix configuration may enhance the flexibility of the sleeve and the ability of the sleeve to be in a compressed, low profile state during delivery. Other sleeve configurations are contemplated.

Preferably, the biodegradable and biocompatible materials are applied with the stent segments 110, 120, 130 in a partially expanded state. For example, THORALON is preferably applied with the stent segments being about 65% to about 95% expanded, and more preferably, from about 75% to about 85% expanded. Applying the materials when the stent segments are in their fully expanded state may prevent the material from compressing down to a sufficiently low profile during delivery. Conversely, applying the materials when the stent segments are in their fully compressed state may cause the material to fracture and break off when the stent segments are fully expanded.

Generally speaking, the above biodegradable and biocompatible materials may be applied onto the segmented stent structure 100 using any method known to one of ordinary skill in the art, including spraying and dip coating. Spraying is preferably conducted by first masking the regions of the stents that are not desired to be coated with the biodegradable/biocompatible material. After the masking is achieved, the biodegradable/biocompatible material is mixed within a solvent such as chloroform, methylene chloride or dimethylacetamide. The biodegradable/biocompatible solution is then heated by any known heating means, such as heating lamps. The solution is then sprayed onto the unmasked regions of the stent surface. As the heat causes the solvent to evaporate whereupon the polymeric biodegradable/biocompatible material hardens, thereby adhering to the surface of the stent.

Because the embodiments shown in Figures 1-3B contemplate multiple stent segments being implanted simultaneously along the length of a stricture, the length of each stent segment may be smaller than that of typical stents used individually. Shorter length stent segments may be able to withstand stresses and strains from the walls of the body lumen and surrounding skeletal muscles better than a single, longer stent. The improved ability to withstand stresses and strains may reduce the probability that the struts of the stent segments will break. For example, implantation of a single stent within the SFA typically would require that the single stent have a length ranging from about 40 mm to about 200 mm. The SFA is an artery located in the leg that undergoes frequent bending and compression as the legs are bent. A single, longer stent may be prone to kinking and potential breakage of its struts as the unstented portions of the SFA bend relatively more than the stented SFA region. The single, longer stent may .not have the flexibility to conform to the continuously changing artery bends. On the other hand, the segmented stent system 100 may contain between about 10 to about 15 stent segments that each have a length of about 15 mm in order to cover the desired stricture within the SFA. Each stent segment may be spaced about 2 mm apart to ensure that the ends do not touch and hit each other. The relatively shorter stent segments are sufficiently spaced apart such that the segments may be better adapted to conform to the continuous changing arterial bends. Additionally, the shorter stent segments may have less tendency to twist. Although Figure 1 shows each of the stent segments 110, 120, 130 having the same longitudinal length, they may have different longitudinal lengths depending on the stricture that the segmented stent structure 100 is implanted within.

The interconnectors 111, 112, 140, 118, 119, 150 may permit movement of the stent segments 110, 120, 130 relative to each other, which renders the entire segmented stent structure 100 flexible. Accordingly, such flexibility may enhance the lateral, longitudinal and torsional flexibility of the entire segmented stent structure 100. When the biodegradable interconnectors, 112, 140, 118, 119, 150 dissolve after implantation, the resultant stented region may be even more flexible, which is advantageous in the continuously bending SFA region.

Traditionally, stenting a relatively long stricture would require implanting a stent that is longer than typical stents. However, longer stents do not possess the desired flexibility needed in many arteries, such as the SFA. In order to overcome the poor flexibility encountered with a longer stent, multiple stents were used. However, the multiple stents were overlapped with each other in order to prevent gaps between the spaced apart stents where restenosis could potentially occur. The overlapped ends often caused kinking of the ends and potential damage of the struts of the stent segments which can, in turn, damage the adjacent artery wall and consequential arterial stenosis. The segmented stent structure 100 may overcome these problems by placing anti-restenosls drugs within the interconnectors 111, 112, 118, 119, 140, 150. When the stent structure 100 is implanted within a body lumen, the anti-restenosis drugs diffuse into the gaps between the stent segments 110, 120, 130 to prevent the onset of restenosis. Suitable anti-restenosis bioactives or antiproliferatives include, but are not limited to, paclitaxel or other taxane derivatives (such as QP-2), actinomycin, methothrexate, angiopeptin, vincristine, mitomycine, statins, C MYC antisense, ABT-578, RestenASE, Resten-NG, 2-chloro-deoxyadenosine, and PCNA ribozyme. Any single antiproliferative or combination thereof may be used. Desirably, the antiproliferative is paclitaxel (commercially available as Taxol®) or a derivative thereof. The paclitaxel is desirably amorphous or dihydrate paclitaxel. Paclitaxel is a natural diterpenoid originally isolated from the bark of the Pacific Yew Tree. Paclitaxel may be used to prevent restenosis by preventing chronic inflammation (by preventing the division of affected cells by stabilizing the microtubule function) and by preventing cell migration (by preventing cells with destructive potential from migrating and accumulating at the injured site). The antiproliferatives may be released into the gaps at a predetermined time at a predetermined rate by loading the antiproliferatives within drug-eluting matrix polymeric materials described in U.S. Patent Nos. 5,380,299, 6,530,951, 6,774,278 and U.S. Patent Application Serial Nos. 10/218,305, 10/223,415, 10/410,587, 10/000,659, and 10/618,977.

Additionally, the interconnectors 111, 112, 140, 118, 119, 150 may be loaded with endothelium-growth-promoting agents that accelerate the growth of endothelial lining around the struts of the stent segments 110, 120, 130, thereby preventing thrombosis of the blood as it encounters the segmented stent structure 100. Suitable endothelial agents that promote healing and re- endothelialization include but are not limited to BCP671, VEGF, estradiols (such as 17-beta estradiol (estrogen)), NO donors, EPC antibodies, biorest, ECs, CD-34 antibodies, and advanced coatings. Rapid endothelialization along the inner surfaces of the stent segments may be beneficial when the stent segments are implanted within the SFA. Mechanical stresses in the SFA cause stent movements relative to the nearby arterial wall that encourages thrombosis and smooth muscle tissue growth. These may be overcome by using a very flexible endovascular device with an inner surface that promotes rapid stent endothelialization to counter the biological effects of motion and microtrauma.

Suitable thrombin inhibitors and anti-thrombogenic agents include, but are not limited to heparin, covalent heparin, or another thrombin inhibitor, hirudin, hirulog, argatroban, D-phenylalanyl-L-poly-Larginyl chloromethyl ketone, or another antithrombogenic agent.

The bioactive may also include anti-inflammatories. Suitable anti-inflammatory/immunomodulators include, but are not limited to dexamethasone, m-prednisolone, interferon g-1b, leflunomide, sirolimus, tacrolimus, everolimus, pimecrolimus, biolimus (such as Biolimus A7 or A9) mycophenolic acid, mizoribine, cycloporine, tranilast, and viral proteins. The bioactive may also include any combination of anti-thrombocytics, anti-inflammatories, antiproliferative agents, and endothelial agents.

In addition to deploying a segmented stent structure, as has been described in great detail, there is also a need to be able to deploy multiple stents at various implantation sites. This is particularly advantageous when the true length of the stent required to cover the stricture is not well identified during a particular procedure. For example, after deploying a stent at a first target site, a physician may not realize until during the stenting procedure that another target site requires implantation of a stent. At this stage in the medical procedure, the withdrawal and insertion of a new delivery device may significantly increase procedure time, patient trauma, and potentially dislodge the stent that has already been implanted. Delivering multiple stents from a single delivery device is also beneficial when a stent segment inadvertently over expands and causes tissue of the body lumen to tear. Inserting of another stent segment using another delivery device can dislodge or disrupt previously placed stent segments. Figure 4 addresses these problems. It shows a multiple stent delivery device 400 that can deliver multiple stents at the same or different treatment sites without having to withdraw and insert another delivery device.

The self-expanding stents 410 are configured in linear order around inner catheter 440. A restraining sheath 420 is disposed over the inner catheter 440. The restraining sheath 420 is an outer tubular member that is coaxial with the inner catheter 440. The restraining sheath 420 maintains the self-expanding stents 410 in compression during delivery. Deployment of a desired number of stents 410 may be accomplished by retracting the distal end of the restraining sheath 420 relative to the inner catheter 440. This will produce one or more self-expanding stents 410 that are entirely exposed.

Still referring to Figure 4, delivery device 400 contains spacer elements 430 which are affixed to an outer surface of the inner catheter 440. Each of the spacer elements 430 are situated between an adjacent pair of stents 410. The spacer elements 430 have a depth sufficient to prevent contact of the adjacent stent segments 410 during proximal movement of the restraining sheath 420 relative to the inner catheter 440. Spacer elements 430 also maintain the self-expanding stents 410 spaced apart at predetermined distances, preventing the stents 410 from axially moving along the outer surface of the inner catheter 440 during delivery. Self-expanding stents 410 may be of various lengths within the delivery device 400. Suitable longitudinal lengths 450 of the spacer elements 430 and suitable longitudinal lengths of the self-expanding stents 410 are dependent upon numerous factors including the length and shape of the stricture to be stented.

Various types of spacer elements 430 are contemplated. For example, the spacer elements 430 may be plastic washers or rings that may be affixed to the inner walls of the catheter 440 by any method known to one of ordinary skill in the art, including gluing. Alternatively, the spacer elements 430 may be molded into the inner catheter 440 as notches or knobs. The washers and notches/knobs may completely extend around a circumference of the outer surface of the inner catheter 440, as shown in Figure 4. Alternatively, the spacer elements 430 may be segmented portions. Each segmented portion may extend only partially around a circumference of the outer surface of the inner catheter 440. Whether the spacer elements 430 completely or partially extend around a circumference of the inner catheter 440, they possess a depth sufficient to prevent contact of the adjacent stent segments 410 during proximal movement of the restraining sheath 420 relative to the inner catheter 440.

In addition to utilizing biodegradable interconnectors to interconnect multiple segmented stents as described above, biodegardable interconnectors may also be used to provide structural support to stents to prevent the stent from moving in undesirable directions during deployment. After successful deployment, the biodegradable interconnectors may biodegrade or dissolve, thereby leaving intact fewer interconnectors to create a stent structure sufficiently flexible within the vessel to withstand loads of the vessel as it bends, compresses, and stretches.

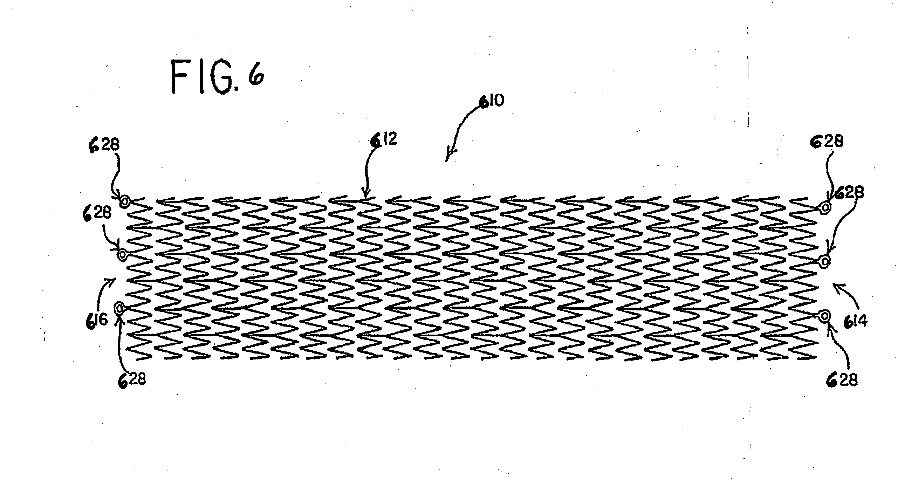

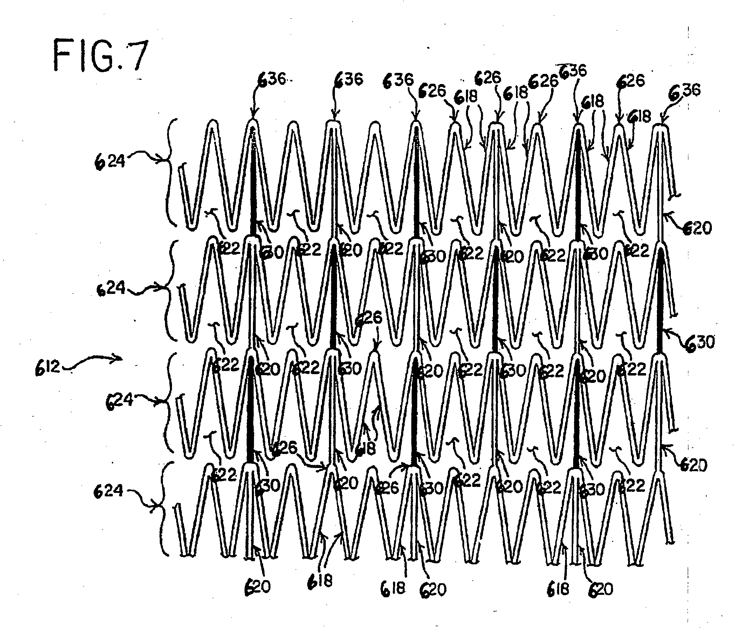

Referring now to the Figure 6, another endovascular stent 610 is shown. The stent 610 is made of a support structure 612 that extends from a first structural end 614 to a second structural end 616. As shown in Figures 7-10, the support structure 612 generally includes a number of interconnecting members 618, 620 that form a series of openings 622 that extend radially through the support structure 612. The openings 622, as well as the flexibility of the interconnecting members 618, 620, allow the stent 610 to expand and compress between a larger expanded diameter and a smaller collapsed diameter. The interconnecting members 618, 620 may be arranged in numerous ways to achieve an expandable support structure, and the examples shown herein are only exemplary. The support structure 612 may be manufactured in numerous ways. For example, the support structure 612 may be made from multiple, discrete components, such as wires that form interconnecting members connected together by welding or other techniques. The support structure 612 may also be made from a unitary structure in which most or all of the interconnecting members are integral without joints between the interconnecting members. One preferred method for making the support structure 612 of the stent 610 is to cut the interconnecting members 618, 620 and openings 622 from a cannula with a laser. Although various materials may be used to construct the stent 610, it is desirable for the stent 610 to be made from a non-biodegradable material to provide long-term intraluminal support. In particular, it is preferred that the stent 610 be made from Nitinol.

As shown, the interconnecting members 618, 620 may include a series of undulating rings 624 that wrap circumferentially around the stent 610. The undulating rings 624 are formed from a series of angular interconnecting members 618 that are connected together by a series of bends 626. The undulating rings 624 may be connected together by longitudinal interconnecting members 620 to form the entire length of the support structure 612 of the stent 610. If desired, the support structure 612 may include integral eyelets 628 at the first and second structural ends 614, 616 for radiopaque markers. Other components and features may also be included with the stent 610.

As shown in more detail in Figure 7, the undulating rings 624 may be arranged in phase with each other. As a result, each longitudinal interconnecting member 620 connects to opposite sides of like features on adjacent undulating rings 624. For example, as shown, one side of a longitudinal interconnecting member 620 may be connected to an undulating ring 624 on the inside of a bend 626 formed between the angular interconnecting members 618. Thus, the angular interconnecting members 618 which form the bend 626 extend acutely from the end of the longitudinal interconnecting member 620. On the other end of the longitudinal interconnecting member 620, the longitudinal interconnecting member 620 may be connected to the outside of a bend 626 formed on an adjacent undulating ring 624. Thus, the angular interconnecting members 618 forming the bend 626 extend obtusely from the end of the longitudinal interconnecting member 620. The described support structure 612 for the stent 610 is only one example of the many types of support structures that are possible. Although the described arrangement of interconnecting members 618, 620 is particularly useful, other support structures may also be used, if desired. For example, although the preferred embodiment employs angular interconnecting members 618 and longitudinal interconnecting members 620, the interconnecting members could also be curvilinear. Other structures that do not use undulating rings 624 could also be used, if desired.

When the above described stent 10 is used to treat some body lumens, fatigue life and proper deployment may become issues of concern. One particularly challenging treatment site is the superficial femoral artery (SFA). Because the SFA continuously changes length and experiences repeated bending, the fatigue life of the stent 610 may become particularly important to ensure adequate performance of the stent 610. One way to increase the fatigue life of a stent 610 is to reduce the number of longitudinal interconnecting members 620. For example, in the stent 610 shown in Figure 6, it may be typical to include four longitudinal interconnecting members 620 between adjacent undulating rings 624. This design has proven successful in treating various intraluminal conditions. However, in the case of the SFA, it may be desirable to reduce the number of longitudinal interconnecting members 620 between adjacent undulating rings 624 from four to three. It may also be beneficial to reduce the number of longitudinal interconnecting member 620 even more to only two longitudinal interconnecting members 620 between adjacent undulating rings 624, if desired. Reducing the number of longitudinal interconnecting members 620 between adjacent undulating rings 624 increases the lengthwise (axial) flexibility of the stent 610. In particular, with fewer longitudinal interconnecting members 620, the circumferential span of each undulating ring 624 which is unconnected to an adjacent undulating ring 624 is greater. As a result, the unconnected portions of the undulating rings 624 are able to flex lengthwise to give the stent 610 greater axial flexibility.

However, one problem with reducing the number of longitudinal interconnecting members 620 is that the stent 610 may become less stable during deployment of the stent 610. In particular, the unconnected portions of the undulating rings 624 may have a greater tendency of flop around or otherwise move in undesirable directions during deployment. As a result, the stent 610 may not deploy in a uniform manner. For example, some of the undulating rings 624 may not wrap uniformly around the inner lumen of the treatment site after deployment. Instead, some portions of the undulating rings 624 may be pulled proximally or distally towards an adjacent undulating ring 624 and away from the opposing undulating ring 624. This is undesirable because it may reduce the effectiveness of the stent treatment.

As shown in Figures 7 through 10, biodegradable connectors 630, 632, 634, 638 may be used to improve the stability of the stent 610 during deployment. Biodegradable materials can be degraded and absorbed by the body over time. The biodegradable connectors may be made from a variety of biodegradable polymers, including the above-listed biodegradable materials from which interconnectors 111, 112, 118, 140, 119 and 150 may be formed.

The biodegradable connectors 630, 632, 634, 638 may be arranged within the support structure 612 in a variety of ways. For example, as shown in Figure 2, biodegradable connectors 630 are longitudinally aligned with adjacent longitudinal interconnecting members 620. The biodegradable connectors 630 are also circumferentially spaced apart and aligned with adjacent longitudinal interconnecting members 620 which are connected to the same undulating rings 624. As a result, a plurality of continuous longitudinal support lines 636 are formed extending along the length of the stent 10. Each longitudinal support line 636 consists of alternating biodegradable connectors 630 and non-biodegradable longitudinal interconnecting members 620 (i.e., non-biodegradable interconnecting member 620-biodegradable connector 630-non-biodegradable interconnecting member 620-biodegradable connector 630).

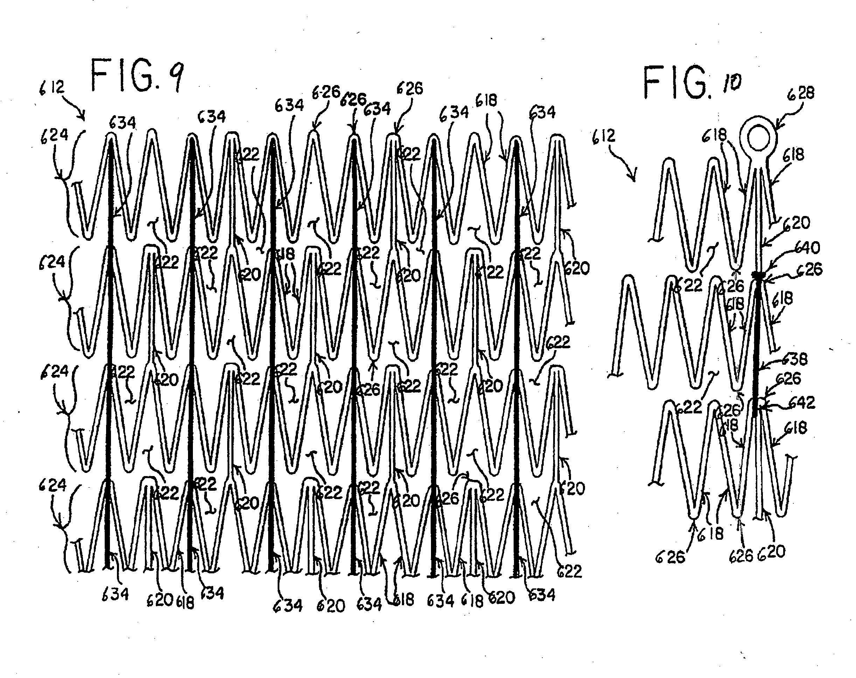

The biodegradable connectors 630, 632, 634, 638 may be attached to the undulating rings 624 in various ways that are well known to those skilled in the art. For example, if biodegradable sutures are used, the sutures may be tied at each end to adjacent undulating rings 624. An example of a suture 638 tied to the undulating rings 624 is shown in Figure 10. As shown, one end 640 of the suture 638 may be tied to a longitudinal interconnecting member 620 adjacent a bend 626 where the angular interconnecting members 618 extend obtusely from the end of the longitudinal interconnecting member 620. The other end 642 of the suture 638 may be tied within a bend 26 formed by an adjacent longitudinal interconnecting member 620 and an angular interconnecting member 18 extending acutely therefrom. This may be accomplished by tying the suture 638 to the bend 626 itself, to the angular interconnecting member 618, or to the longitudinal interconnecting member 620. A suture 638 may be a useful biodegradable connector 630, 632, 634, 638 in the support structure 612 shown in Figure 7 because the biodegradable connectors 630 will primarily experience tension forces and less compressive forces. Rigid or semi-rigid biodegradable connectors 630, 632, 634, 638 may also be used. For example, strips may be used in place of sutures. The strips may be attached to the support structure 612 by molding the strips onto the support structure 612, gluing the strips to the support structure 612, or heat bonding or by other known techniques. Preferably, the width of the biodegradable connector 630, 632, 634, 638 is 010" or less. A thin biodegradable connector 630, 632, 634, 638 is desirable so that the biodegradable connectors 630, 632, 634, 638 do not interfere with the support structure 612 of the stent 610 when it is collapsed. Thus, it is preferred that the stent 610 be capable of collapsing to the same or similar low profile that would be possible without the presence of the biodegradable connectors 630, 632, 634, 638. However, a slightly larger collapsed configuration may be acceptable due to the increased deployment stability of the stent 610.

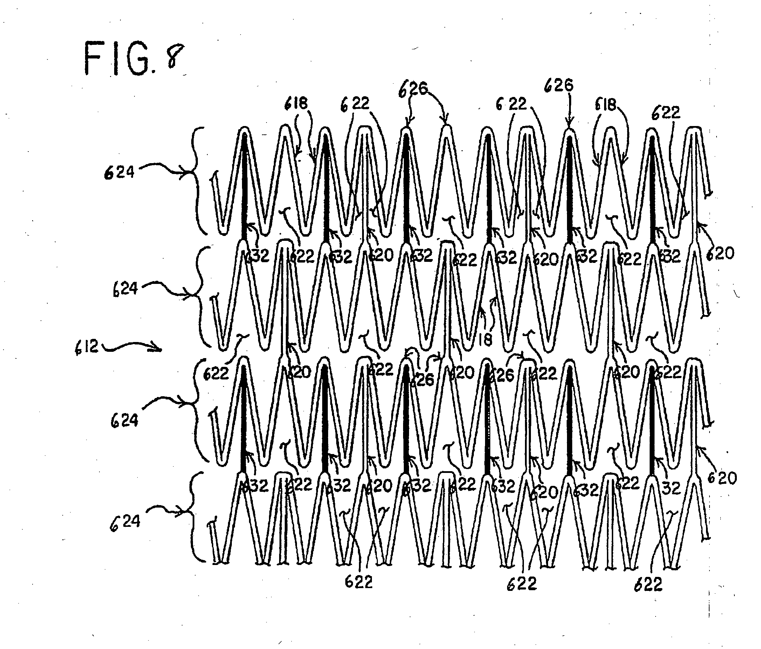

Turning to Figure 8, the biodegradable connectors 632 may be circumferentially offset from longitudinally adjacent longitudinal interconnecting members 620. Thus, an opening 622 is adjacent each end of the biodegradable connectors 632 between the undulating rings 624 the biodegradable connectors 632 are connected to and the next adjacent undulating rings 624. The biodegradable connectors 632 may also be circumferentially spaced apart and aligned with adjacent longitudinal connecting members 620 if the undulating rings 624 have a constant length. The arrangement shown in Figure 8 may provide the stent 610 with greater flexibility during deployment than the arrangement shown in Figure 7 but still may provide adequate stability to insure uniform deployment.

Turning to Figure 9, another arrangement for the biodegradable connectors 634 is shown. Unlike Figures 7 and 8, the biodegradable connectors 634 in Figure 9 extend along substantially the entire length of the support structure 612 of the stent 610. Each of the biodegradable connectors 634 is preferably positioned between adjacent longitudinal interconnecting members 20 so that the biodegradable connectors 634 do not overlie the longitudinal interconnecting members 620. As shown, the biodegradable connectors 634 cross the undulating rings 624 and extend across the openings 622 between the undulating rings 624. It is preferred that the biodegradable connectors 634 are connected to each undulating ring 624 that is crossed.

The advantages of the stent 610 are now apparent. A stent structure 612 with improved fatigue properties may be provided by reducing the number of interconnecting members 618, 620 used in the stent structure 612. This may be particularly useful for reducing the number of longitudinal interconnecting members 620 between undulating rings 624. Because of the reduced number of interconnecting members 618, 620, the support structure 612 may be more flexible, both in lengthwise stretching and in bending. However, in order to maintain uniform deployment, biodegradable connectors 630, 632, 634, 638 may be used to connect together portions of the support structure 612. It may be particularly helpful to connect adjacent undulating rings 624 together across open areas 622. As a result, the biodegradable connectors 630, 632, 634, 638 restrain movement of the interconnecting members 618, 620 relative to the other interconnecting members 618, 620 in the support structure 612. Thus, the support structure 612 is more stable during deployment. In order to achieve increased flexibility of the stent 610 after implantation, the biodegradable connectors 630, 632, 634, 638 degrade or are absorbed so that eventually only the non-biodegradable portions 618, 620 of the stent 610 remain. The time that it takes for the biodegradable connectors 630, 632, 634, 638 to degrade may be modified as desired by selecting a suitable biodegradable material, altering the mix of biodegradable materials, or changing the thickness, structure or preparation of the biodegradable connectors 630, 632, 634, 638. It may be desirable for the biodegradable connectors 630, 632, 634, 638 to degrade within hours or days after deployment since one advantageous use of the biodegradable connectors 630, 632, 634, 638 is to improve the uniformity of stent 610 deployment but degrade after deployment to provide improved stent 610 flexibility. Accordingly, after the biodegradable connectors 630, 632, 634, 638 degrade, a single stent structure 612 made from non-biodegradable interconnecting members 618, 620 remains implanted to provide long-term intraluminal support. Thus, it may be possible to treat an organ, such as the SFA, with only one stent 610 instead of multiple stents.

Another means to address the concerns of lack of stability during deployment of an expandable stent structure will now be discussed in which stabilizing elements are added to the stent framework.

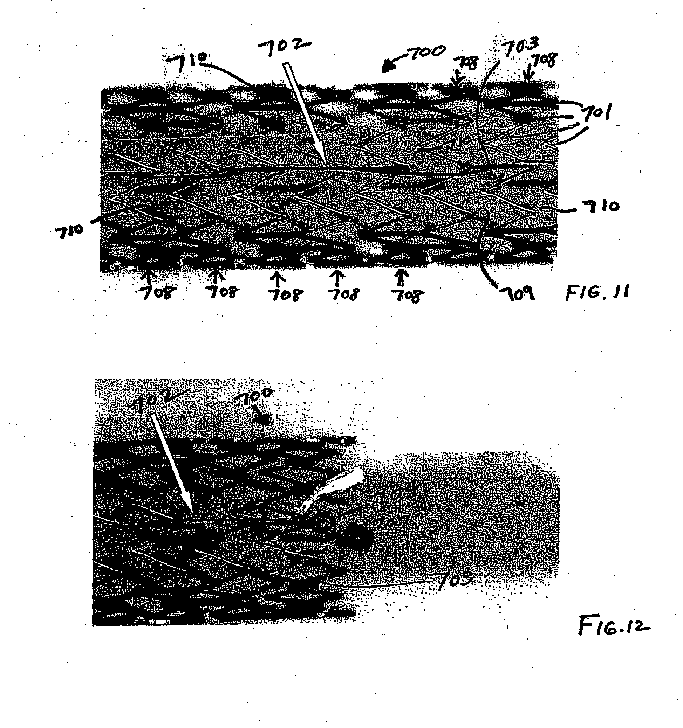

Referring now to the figures, and particularly to Figure 11, an intraluminal stent 700 is shown. The stent 700 comprises a generally cylindrical body formed from a series of struts 701 that circumferentially extend in a zigzag pattern. Each of the zigzag patterns 708 may be connected by interconnecting members 703 to form the entire longitudinal length of the stent 700. The zigzag patterns 708 and two interconnecting members 703 form an enclosed region known as a unit cell 710. Each of the unit cells 710 comprises an opening which allows the stent 700 to self-expand and compress between a larger expanded diameter and a smaller collapsed diameter. The unit cells 710 span the entire longitudinal and circumferential length of the stent 700. The struts 701 may be configured in numerous ways to achieve a self-expandable structure, and the examples shown herein are only exemplary. The stent 700 may be manufactured in numerous ways. In a preferred manufacturing method, the stent 700 is laser cut from a cannula. Although various materials may be used to construct the stent 700, it is preferred that the stent 700 be made from a non-biodegradable material to provide long-term intraluminal support. In particular, it is preferred that the stent 700 be made from Nitinol. Radiopaque markers may be affixed along the struts 701. Other components and features may also be included with the stent 700.

The stent 700 as shown in Figure 11 is designed with a minimum number of interconnecting members 703. The minimum number of interconnecting members 703 provides the stent 700 with the ability to flex lengthwise as well as bend and twist within tortuous body lumens, such as the superficial femoral artery (SFA), which is continuously bending, stretching, and compressing. However, one problem with adding such increased axial and torsional flexibility to the stent 700 is that it may be difficult to load and deploy. The stent 700 may become less stable during deployment of the stent 700. In particular, the unconnected portions of the zigzag patterns 708 may have a greater tendency to move in undesirable directions and overstretch in the longitudinal direction during deployment when an outer sheath is pulled back to expose the stent 700. Additionally, the loading of the stent 700 between an inner sheath and the outer sheath may be problematic as the stent 700 may lack sufficient rigidity to adequately be compressed and loaded therebetween. When the distal end of the stent 700 expands to contact the wall of the body lumen, any axial movement of the delivery system may stretch the unit cells 710 of the stent 700 as they are deployed to create non-uniform expansion of the unit cells 710. Non-uniform expansion of the unit cells 710 may lead to non-uniform support of the vessel and potential non-uniform elution of a bioactive material if the struts 701 are coated with a bioactive material.

The stent 700 has been imparted with axial (i.e., longitudinal) stiffness by a stabilizing element 702 to overcome the loading and deployment problems associated with the flexibility of the stent 700. Figures 11 and 12 show a fixed-length suture 702 connected to both supporting ends of the stent 700. Figure 12 shows that a first end 703 of the suture 702 is tied around the eyelet 704 at the first supporting end 707 of the stent 700. A second end of the suture 702 may be connected at the second supporting end of the stent 700. The suture 702 extends along the struts 701 from the first supporting end 707 to the second end of the stent 700 to provide sufficient axial stiffness and rigidity to the stent 700 structure during loading and deployment of the stent 700. In particular, the suture 702 will experience tensile forces and acts as a frictional member between the zigzag patterns 708 to restrict undesirable longitudinal stretching of the stent 700 during deployment. The suture 702 also provides sufficient stability so as to load the stent 700 within a sheath. Figures 11 and 12 show that the suture 702 interweaves in an "over-and-under" pattern through the struts 701 and the interconnecting members 703 extending substantially parallel to a longitudinal axis of the cylindrical body of the stent 700.

The suture 703 may comprise various sizes. In the example of Figures 11 and 12, the suture 702 has a diameter of about .07 mm. Such a diameter is sufficient for the suture 702 to loosely interweave through the struts 701 without obstructing the lumen 709 of the stent 700. Other patterns of the suture 702 besides interweaving through the struts 701 are contemplated. For example, the suture 702 may extend along an outer surface or inner surface of the struts 701.

The suture 703 may be biodegradable or non-biodegradable. In particular, it is preferred that the suture 702 is formed from a biodegradable material so that the suture 702 biodegrades or dissolves after a predetermined time within the body lumen. After the stent 700 is loaded and deployed, the axial stiffness imparted by suture 702 is no longer needed. Having a dissolvable suture 702 enables the stent 700 structure to improve its axial flexibility, which is a desirable characteristic for tortuous body vessels such as the SFA. The biodegradable material may be a copolymer of glycolide and L-lactide, also known as VICRYL. VICRYL biodegrades after a period of time, thereby leaving the stent 700 unconstrained within the body lumen. Various blends of polyglycolic acid, lactic acid or caprolactone may be used to make the suture 702 biodegradable. Other types of biodegradable or absorbable sutures are contemplated which are formed from synthetic polymer fibers and that are known to one of ordinary skill in the art. Yet further types of biodegradable materials may be utilized including the above-listed biodegradable materials from which interconnectors 111, 112, 118, 140, 119 and 150 may be formed from. Additionally, the absorbable sutures may be braided or made from a monofilament.

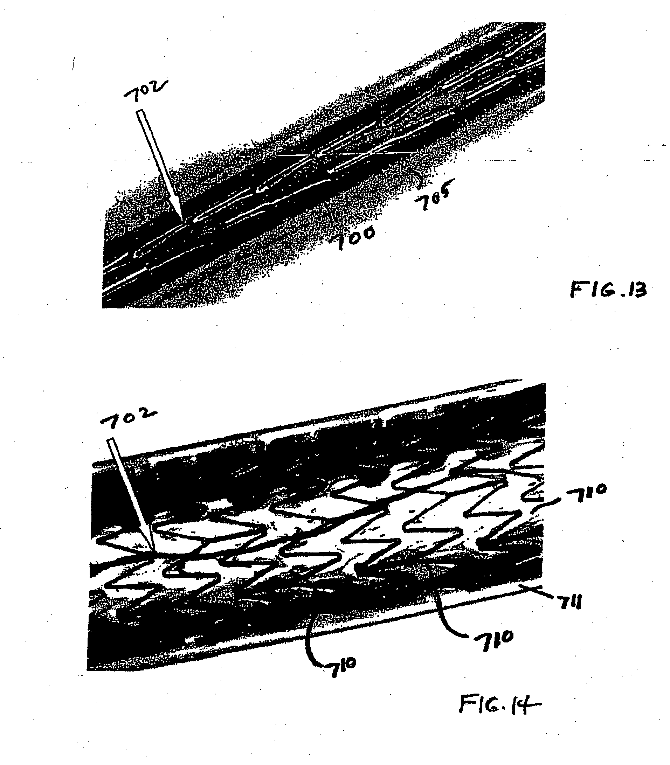

Nonbiodegradable (i.e., biostable) materials may also be used such as artificial fibers, like polypropylene, polyester or nylon. Other types of biostable materials may also be used, including the above-list of biostable materials from which sleeve 510 may be formed from. Having the suture 702 remain intact and disposed on the stent 700 may not interfere with ability of the stent 700 to radial expand in vivo, as shown in Figure 14. Figure 14 shows the stent 700 after deployment. The suture 702 is shown as interweaving in-and-out of the unit cells 710 without restricting the stent 700 from radially expanding within a simulated body vessel 711 (Figure 14). The flexibility of the suture 702 allows the stent 700 to expand and compress between a larger expanded diameter and a smaller collapsed diameter. The suture 702 may provide axial stiffness, and optionally torsional stiffness, to the stent 700 during loading and deployment without interfering with expansion (Figure 14) and compression (Figure 13) of the stent 700 in vivo, as shown in Figure 13. Figure 13 shows that the suture 702 does not restrict the ability of the stent 700 to compress and be loaded into a sheath 705.

More than one suture may be used to impart the desired axial stiffness to the stent 700. In particular, it is preferred that two fixed-length sutures may be used. Each of the sutures extend the entire longitudinal length of the stent 700 and are circumferentially spaced apart from each other. In one example, each of the two sutures may be spaced apart about 180°.

The sutures may be attached to the struts 701 at both ends of the stent 700 in various ways that are well known to those skilled in the art. For example, each of the ends of the sutures may be tied off at an eyelet 704. An example of an end of a suture 702 tied to an eyelet 704 is shown in Figure 12. Alternatively, the ends of each of the sutures may be bonded to the struts 701 of the stent 700 with a biologically-derived or absorbable adhesive. The suture 702 may be attached anywhere near the end cells 710 of the stent 700 to control the longitudinal length of the stent 700 (i.e., prevent substantial overstretching) when the stent 700 is radially expanding. The suture 702 may be a useful axial and/or torsional stiffener in the stent 700 as shown in Figures 11 and 12 because the suture 702 will primarily experience tension forces and less compressive forces.

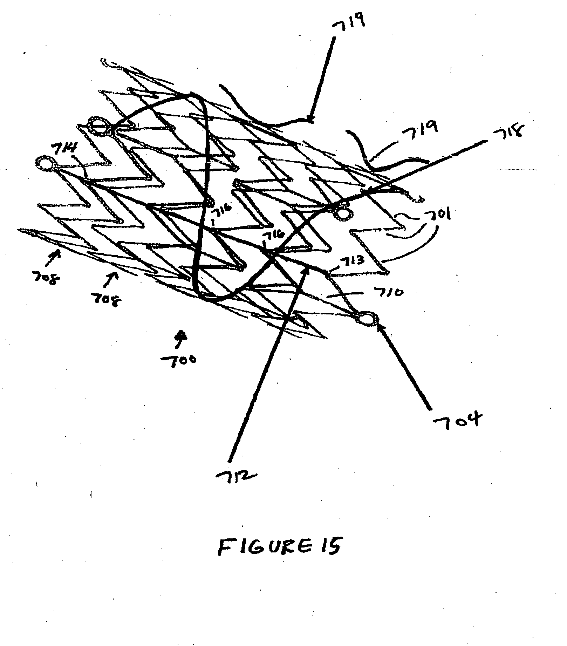

In addition to having sutures that extend substantially linearly along the longitudinal axis, some of the sutures may extend about the stent 700 in a helical manner to impart a combination of torsional rigidity and axial rigidity to the stent 700. Torsional rigidity may substantially reduce the likelihood that the stent 700 will undesirably bend or twist during loading and deployment of the stent 700. The number of windings of the suture per unit length will determine the extent to which axial rigidity is achieved. Having a fraction of the sutures extend substantially linearly along the stent 700 as shown in Figures 11 and 12, and a fraction of the sutures extend helically (Figure 15) offers both axial and torsional rigidity during loading and deployment.

Determining whether to configure the sutures 702 along the struts 701 of the stent 700 in a longitudinal configuration or a helical configuration is dependent upon several factors, including the relative stiffness of the suture material being utilized. As an example, if the suture 702 material is relatively stiff, then the suture 702 material may be configured along the stent 700 in the longitudinal direction. However, if the suture 702 material exhibits relatively high flexibility, then the material may be configured to be helically wound in a helical fashion.

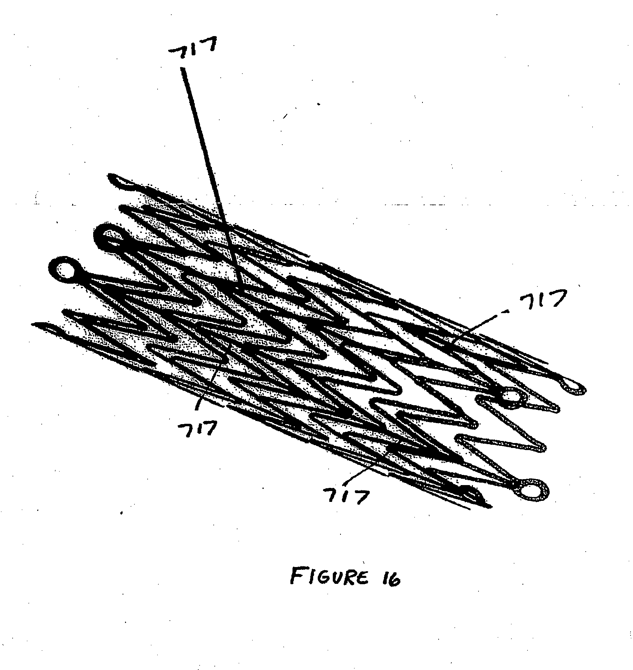

Other means for imparting rigidity to the stent 700 are contemplated. For example, rigid or semi-rigid stabilizing elements may also be used. Strips 712 (Figure 15) may be used in place of sutures, as shown in Figures 15 and 16. The strips 712 may include a variety of rigid or semi-rigid polymeric materials such as ePTFE, PLA, and other materials described above with respect to the biodegradable connectors. In a preferred example, the strips 712 may be formed from THORALON, which is a porous, elastic, biocompatible polyurethane, as described above.

The strips 712 may be attached to the stent 700 in any number of ways, including molding the strips 712 onto the struts 701, gluing the strips 712 to the struts 701, or heat bonding the strips 712 onto the struts 701. In a preferred method of attachment, a spray process may be used to selectively apply strips 712 to the outer diameter of the stent 700 such that the strips 712 traverse the longitudinal length of the stent 700.

The spray process provides the ability to program a variety of shapes, thicknesses, and patterns of material onto the stent 700. The strips 712 may have many forms and connection patterns depending on the structure of the stent 700. Alternative methods of applying the strips 712 of material include dip coating or a masking technique as known in the art.

Preferably, the width of the strips 712 is relatively thin so that the strips 712 do not interfere with the stent 700 when it is collapsed. Thus, it is preferred that the stent 700 be capable of collapsing to the same or similar low profile that would be possible without the presence of the strips 712. However, a slightly larger collapsed configuration may be acceptable due to the increased deployment stability of the stent 700.

Figure 1 5 shows a strip 712 extending longitudinally along the entire length of the stent 700. The strip 712 comprises a first end 713 and a second end 714. The strip 712 connects adjacent unit cells 710 and adjacent interconnecting members 703. The strip 712 may or may not be aligned with the apexes 716 of each of the unit cells 710. The straight cell-to-cell strip configuration of the strip 712 as shown in Figure 15 may limit the axial extension of the stent 700 due to the rigidity of the strip 712 during deployment. Other connection patterns of the strip 712 are contemplated. Design variables that impact the rigidity of the strip 712 include thickness of the strip 712, the number of strips 712, the location of the strip 712 about the stent 700 body, the connection pattern of the strip 712, and the elastic stiffness of the strip 712. The exact combination of such design variables may depend on the particular structural type of stent used and the implantation site.

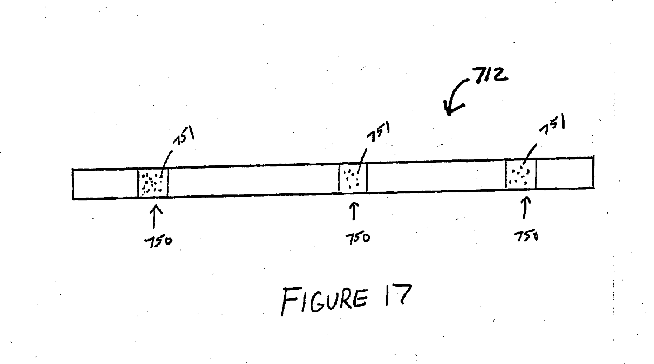

The single strip 712 may be partitioned into segments so as to uniformly distribute the supplemental stiffness about the body of the stent 700. One way of partitioning the strip 712 is to incorporate mechanical frangible zones 750 (i.e., scores) falong selected regions of the strip 712, as shown in Figure 17. Figure 17 shows a strip 712 with three frangible zones 750, each being spaced apart from each other. Each of the frangible zones 750 has geometric discontinuities. The geometric discontinuities may comprise multiple depressions 751 that create fracture planes that break upon radial expansion of the stent 700. The mechanical frangible zones may also be embrittled and/or hardened to create the fracture planes that break upon radial expansion of the stent 700. The frangible zones 750 allow the strip 712 to fracture in a controllable fashion due to the internal force of the self-expanding stent 700. Alternatively, a post-dilation balloon may be inserted into the lumen of the stent 700 and thereafter sufficiently inflated to assist with the fracture of the strip 712 in a controllable fashion. The selective breaking apart of the longitudinal strip 712 into segments enables the strip 712 to become entirely disconnected so as to enable the stent 700 to possess the desired level of axial flexibility in vivo and provide the desired radial force against the vessel walls. The frangible zones 750 may be created such that the strip 712 breaks apart at about every two unit cells 710. The frangible zones 750 may also be designed to create some segments which are longer than others. The exact length of each of the frangible zones 750 and the number of such frangible zones 750 are dependent upon numerous factors, including the structural type of stent 700 used and the extent to which the implanted site of the stent 700 undergoes bending, torsion, lengthening and shortening. Relatively more frangible zones 750 may be desirable in an implanted region such as the SFA which is prone to continuous bending, torsion, lengthening and shortening. Frangible zones 750 may also be designed to break after a relatively small number of repetitive or cyclic loadings (i.e., in-vivo conditions) such that the extreme motion of the vessel fractures and ultimately breaks the strips 712.

As an alternative to a single longitudinal strip 712 having selected frangible zones which traverses the entire longitudinal length of the stent 700, Figure 16 shows that the stent 700 may comprise multiple discrete segments 717 which extend substantially linearly along a portion of the longitudinal length of the stent 700. In the example of Figure 16, four discrete THORALON segments 717 may be applied (e.g., spraying) to the stent 700 in its partially expanded configuration. As the stent 700 self-expands during deployment, the THORLAON segments 717 are tensioned so as to resist axial extension of the stent 700.

The segments 717 may provide sufficient supplemental stiffness to the stent such that it does not fully expand to its fully designed operating diameter. Accordingly, a post dilatation balloon may further radially expand the stent 700 to fracture the strip 712 and render the segments 717 inactive after expansion of the stent 700. Balloon dilatation may often expand a self-expanding stent to its fully designed operating diameter and sometimes as much as about 10% beyond its fully designed operating diameter. The additional expansion of the stent 700 by balloon dilatation may strain the strips 712 sufficiently so as to fracture the strips 712 at pre-determined frangible zones 750.

The segments 717 may be configured in a variety of patterns. The segments 717 may be aligned or offset relative to each other. The length of the segments 717 may vary depending on the stiffness desired. The segments 717 may be circumferentially spaced apart as desired.

One method of applying the THORALON segments 717 as shown in Figure 16 involves a masking technique. For example, the stent 700 may be inserted into a slotted transfer tube. The stent 700 may assume a partially expanded configuration within the slotted transfer tube. THORLAON may then be sprayed through the slots of the transfer tube and thereafter cured for a predetermined time, temperature, and pressure until the THORLAON has reached a sufficient hardness level. The stent 700 may then be withdrawn from the slotted transfer tube. Other rigid or semi-rigid polymeric materials besides THORLAON which act to to supplement the stiffness of the stent 700 may also be used.

The strip 712 may be spiraled about the stent 700 to create a helical strip 718, as shown in Figure 15. The helical strip 718 may provide a combination of axial, radial, and torsional stiffening, thereby providing sufficient stability to the structure of the stent 700 during loading and deployment. The number of windings per unit longitudinal length of the strip 718 may determine the axial stiffening imparted to the stent 700 by helical strip 718. The helical strip 718 may be selectively placed onto the outer diameter of the stent 700 to permit the unit cells 710 to readily close during loading of the stent 700 within the delivery system and readily open during deployment of the stent 700 at the implantation site.

Similar to strip 712, the helical strip 718 may have frangible zones which cause the helical strip 718 to controllably break into helical segments upon deployment of the stent 700, thereby rendering the helical segments inactive after expansion of the stent 700. A post dilatation balloon may further radially expand the stent 700 to fracture the helical strip 718. The helical strip 718 may be applied onto a surface of the stent 700 by utilizing a spiral slotted transfer tube. The strip 718 may be disposed onto the stent 700 at any number of locations, including in the plane of the struts 701, along the outer diameter of the stent 700, and along the inner diameter of the stent 700. The polymeric material may then be sprayed through one or more spiral slots of the transfer tube, and thereafter cured for a predetermined time, temperature, and pressure until the polymeric material has reached a sufficient hardness level. The stent 700 may then be withdrawn from the spiral slotted transfer tube.

In a preferred example, the helical strip 718 is formed from THORALON. Countervailing design attributes of rigidity and flexibility are desirable for the THORALON helical strip 718. On the one hand, it is desirable for the THORALON helical strip 718 to be adequately flexible so as to not fracture as it undergoes tension during the deployment of the stent 700 and not obstruct operation of the stent 700 in vivo. On the other hand, it is desirable for the THORALON helical strip 718 to sufficiently remain rigid to maintain the stent 700 in a stabilized form when the outer sheath is removed during deployment of the stent 700. Numerous design variables affect the THORLAON strip's 718 stiffness and flexibility, including the porosity of the THORLAON and its chemical formulation. One of ordinary skill in the art would be able to select the desired design variables to achieve the desired flexibility and stiffness of the THORLAON strip 718.

Determining whether to place longitudinal strips 712 or helical strips 718 onto the outer diameter of the stent 700 is dependent upon several factors, including the relative stiffness of the reinforcing polymeric material being utilized. As an example, if the polymeric material is relatively stiff, then the polymeric material may be adapted to be configured along the stent 700 as the longitudinal strip 712. However, if the polymeric material exhibits relatively high flexibility, then the material may be adapted to be helically wound to create the helical strip 718.

I.In yet another embodiment, Figure 15 shows two strips 719, each having free ends that are unattached to the struts 701 of the stent 700. As the stent 700 radially expands to contact the vessel wall, the strips 710 frictionally engage with the vessel wall. The strips 719 may provide enough axial and torsional resistance to minimize lengthening and thus significantly reduce non-uniform deployment of the stent 700. As the axial and torsional stiffness of the stent 700 is reduced (i.e.., the flexibility is increased) in order to accommodate the motions of the vasculature, it becomes prone to buckling upon deployment. While the stent 700 is positioned in the deployment system, the sheath and the stent 700 may provide sufficient constraints to buckling. However, upon deployment, when the stent 700 is pushed out of the sheath, frictional forces are imparted to the stent 700 by the sheath and the stent 700 becomes momentarily free of the sheath such that the stent 700 may be prone to buckling. The strips 710 may provide sufficient supplemental axial and/or torsional stiffness such that the undesirable dynamic buckling modes are suppressed, thereby resulting in a more uniform deployment of the stent 700. Strips 719 need not be frangible as they are already partitioned along the outer diameter of the stent 700. The strips 719 may have a roughened surface that is disposed along the stent 700. Alternatively, the strips 719 of material may comprise sharp edges or points. The strips 719 may preferably be biodegradable so as to dissolve after the stent 700 has been deployed within the implanted region. Alternatively, because the strips 719 do not interfere with the ability of the stent 700 to exert radial pressure and flexibly conform to changes in the vessel shape that the stent is implanted within, the strips 719 may be non-biodegradable, passively remaining in vivo along the struts 701.

Stent 700 may have a combination of the above mentioned stabilizing elements (e.g., sutures, strips, and segments) in both longitudinal and helical form to provide the desired axial and torsional stiffness during loading and deployment of the stent 700. The stabilizing elements are preferably biodegradable so as to allow the stent 700 to function as intended (i.e., undergo bending, compression, and stretching in conformance with the movement of the body vessel that it is implanted within).

The advantages of the stent 700 having stabilizing elements are now apparent. Because the structure of the stent 700 is inherently flexible, the stent 700 may require longitudinal and torsional stability during the loading of the stent 700 into a delivery system and during deployment of the stent 700. The stabilizing elements described above in the form of sutures, strips, and segments assist in imparting the required stiffness and rigidity during loading and deployment. After implantation, because such stiffness and rigidity may not be required, the stabilizing elements may biodegrade so as to allow the stent 700 to possess improved flexibility in the vessel. In particular, it may be desirable for such biodegradable stabilizing elements to degrade within hours or days after deployment since one advantageous use of the biodegradable stabilizing elements is to improve the uniformity of stent 700 deployment but thereafter degrade after deployment to provide improved stent 700 flexibility. The removal of the stabilizing elements after the stent 700 has been implanted may also reduce the loads incurred by the struts 701 and interconnectors 703, thereby minimizing the risk of the stent 700 fracturing. The time that it takes for the biodegradable stabilizing elements to degrade may be modified as desired by selecting a suitable biodegradable material, altering the mix of biodegradable materials, or changing the thickness, structure or preparation of the biodegradable stabilizing elements. Accordingly, after the biodegradable stabilizing elements degrade, a single stent structure 700 made from a minimal number of non-biodegradable struts 701 and interconnectors 703 remain implanted to provide long-term intraluminal support.

It should be understood that the stent 700 as shown herein is only one type of stent that may be used with the stabilizing elements. The stabilizing elements as described above may be used on other structural types of stents, such as a stent with undulating rings, or a stent having curvilinear interconnectors.

It should also be understood that all of the stabilizing elements described herein may be arranged in many ways such as helical, linear, and non-linear configurations.

As has been described, biodegradable interconnectors, sleeves, and various stabilizing elements may be utilized separately or in combination to create a stent structure that is flexible enough when implanted yet sufficiently rigid to overcome problems of loading and deployment typically associated with flexible stent structures.

While preferred embodiments of the invention have been described, it should be understood that the invention is not so limited, and modifications may be made without departing from the invention. The scope of the invention is defined by the appended claims, and all devices that come within the meaning of the claims, either literally or by equivalence, are intended to be embraced therein. Furthermore, the advantages described above are not necessarily the only advantages of the invention, and it is not necessarily expected that all of the described advantages will be achieved with every embodiment of the invention.

## Claims

1. A medical device (100) configured for delivery along a body lumen and implantation at a delivery site, the device comprising a plurality of respective stent segments (110, 120, 130) arranged along a common axis, each stent segment (110, 120, 130) having a radially compressed form for delivery and a radially expanded form, there being provided at least one temporary stabilizing element shaped as a polymer strip (111, 112, 118, 119, 140, 150, 200, 201) which is aligned along the longitudinal direction of the stent segments and operatively interconnects at least two of the stent segments (110, 120, 130) to reduce the axial flexibility of the medical device (100) during delivery and implantation, the stabilizing element (111, 112, 118, 119, 140, 150, 200, 201) being adapted to be disconnected after delivery and implantation to increase the flexibility of the device post implantation.

2. A medical device according to Claim 1, wherein each stabilizing element (111, 112, 118, 119, 140, 150, 200, 201) operatively interconnects adjacent stent segments (110, 120, 130).

3. A medical device according to Claim 1, wherein each stabilizing element (200, 201) extends the entire length of the device.

4. A medical device according to any one of claims 1 to 3, in which the at least one temporary stabilizing element (111, 112, 118, 119, 140, 150, 200, 201) comprises a biodegradable element.

5. A medical device according to any one of claims 1 to 3, in which the at least one temporary stabilizing element (111, 112, 118, 119, 140, 150, 200, 201) comprises a frangible element.

6. A method of manufacturing a medical device comprising a plurality of respective stents (110, 120, 130) arranged along a common axis with stabilizing elements (111, 112, 118, 119, 140, 150, 200, 201), comprising the steps of:
(a) inserting a plurality of expandable stents into a transfer tube, the transfer tube comprising one or more slots;
(b) expanding the stents partially within the slotted transfer tube;
(c) spraying a semi-rigid or rigid polymeric material through the one or more slots onto an outer surface of a plurality of struts of the stents; and
(d) curing the sprayed outer surface.

7. The method of manufacture of claim 6, wherein the curing is achieved at a predetermined temperature and pressure for a predetermined time.

8. The method of manufacture of claim 6, wherein the polymeric material comprises THORALON.

9. The method of manufacture of claim 6, further comprising the step of: (e) withdrawing the stents (110, 120, 130) from the slotted transfer tube.

10. The method of manufacture of claim 6, further comprising the step of:
(e) creating a frangible zone along the sprayed outer surface.

## Patentansprüche

1. Medizinische Vorrichtung (100), die zur Zufuhr entlang einem Körperlumen und zur Implantierung an einem Zufuhrort konfiguriert ist, wobei die Vorrichtung mehrere jeweilige entlang einer gemeinsamen Achse angeordnete Stentsegmente (110, 120, 130) umfasst, wobei jedes Stentsegment (110, 120, 130) eine radial zusammengedrückte Form für die Zufuhr und eine radial expandierte Form hat, wobei mindestens ein vorläufiges Stabilisierungselement vorgesehen ist, das als ein Polymerstreifen (111, 112, 118, 119, 140, 150, 200, 201) gestaltet ist, entlang der Längsrichtung der Stentsegmente ausgerichtet ist und mindestens zwei der Stentsegmente (110, 120, 130) miteinander wirkverbindet, um die axiale Flexibilität der medizinischen Vorrichtung (100) während der Zufuhr und der Implantierung zu reduzieren, wobei das Stabilisierungselement (111, 112, 118, 119, 140, 150, 200, 201) geeignet ist, nach der Zufuhr und der Implantierung abgetrennt zu werden, um die Flexibilität der Vorrichtung nach der Implantierung zu erhöhen.

2. Medizinische Vorrichtung nach Anspruch 1, wobei jedes Stabilisierungselement (111, 112, 118, 119, 140, 150, 200, 201) benachbarte Stentsegmente (110, 120, 130) miteinander wirkverbindet.

3. Medizinische Vorrichtung nach Anspruch 1, wobei sich jedes Stabilisierungselement (200, 201) entlang der gesamten Länge der Vorrichtung erstreckt.

4. Medizinische Vorrichtung nach einem der Ansprüche 1 bis 3, wobei das mindestens eine vorläufige Stabilisierungselement (111, 112, 118, 119, 140, 150, 200, 201) ein biologisch abbaubares Element umfasst.

5. Medizinische Vorrichtung nach einem der Ansprüche 1 bis 3, bei der das mindestens eine Stabilisierungselement (111, 112, 118, 119, 140, 150, 200, 201) ein zerbrechliches Element umfasst.

6. Verfahren zur Herstellung einer medizinischen Vorrichtung, die mehrere jeweilige Stents (110, 120, 130) umfasst, die entlang einer gemeinsamen Achse mit Stabilisierungselementen (111, 112, 118, 119, 140, 150, 200, 201) angeordnet sind, umfassend die folgenden Schritte:
(a) Einführen mehrerer expandierbarer Stents in eine Transferröhre, die einen oder mehrere Schlitze umfasst,
(b) partielles Expandieren der Stents in der geschlitzten Transferröhre,
(c) Sprühen eines halbstarren oder starren polymeren Materials durch den einen oder die mehreren Schlitze auf eine äußere Oberfläche von mehreren Verstrebungen der Stents und
(d) Aushärten der besprühten äußeren Oberfläche.

7. Herstellungsverfahren nach Anspruch 6, wobei das Aushärten bei einer vorbestimmten Temperatur und einem vorbestimmten Druck für eine vorbestimmte Dauer erzielt wird.

8. Herstellungsverfahren nach Anspruch 6, wobei das polymere Material THORALON umfasst.

9. Herstellungsverfahren nach Anspruch 6, ferner umfassend den Schritt
(e) Herausziehen der Stents (110, 120, 130) aus der geschlitzten Transferröhre.

10. Herstellungsverfahren nach Anspruch 6, ferner umfassend den Schritt
(e) Schaffen einer zerbrechlichen Zone entlang der besprühten äußeren Oberfläche.

## Revendications

1. Dispositif médical (100) configuré pour être acheminé le long d'une lumière corporelle et implanté au niveau d'un site de distribution, le dispositif comprenant une pluralité de segments de stent respectifs (110, 120, 130) agencés le long d'un axe commun, chaque segment de stent (110, 120, 130) ayant une forme comprimée radialement pour l'acheminement et une forme déployée radialement, au moins un élément de stabilisation temporaire en forme de bande polymère (111, 112, 118, 119, 140, 150, 200, 201) étant prévu, lequel est aligné avec la direction longitudinale des segments de stent et relie entre eux fonctionnellement au moins deux des segments de stent (110, 120, 130) pour réduire la flexibilité axiale du dispositif médical (100) au cours de l'acheminement et de l'implantation, l'élément de stabilisation (111, 112, 118, 119, 140, 150, 200, 201) étant prévu pour être déconnecté après l'acheminement et l'implantation afin d'augmenter la flexibilité du dispositif après l'implantation.

2. Dispositif médical selon la revendication 1, dans lequel chaque élément de stabilisation (111, 112, 118, 119, 140, 150 200, 201) relie entre eux fonctionnellement des segments de stent adjacents (110, 120, 130).

3. Dispositif médical selon la revendication 1, dans lequel chaque élément de stabilisation (200, 201) s'étend sur toute la longueur du dispositif.

4. Dispositif médical selon l'une quelconque des revendications 1 à 3, dans lequel l'au moins un élément de stabilisation temporaire (111, 112, 118, 119, 140, 150, 200, 201) comprend un élément biodégradable.

5. Dispositif médical selon l'une quelconque des revendications 1 à 3, dans lequel l'au moins un élément de stabilisation temporaire (111, 112, 118, 119, 140, 150, 200, 201) comprend un élément frangible.

6. Procédé de fabrication d'un dispositif médical comprenant une pluralité de stents respectifs (110, 120, 130) agencés le long d'un axe commun avec des éléments de stabilisation (111, 112, 118, 119, 140, 150, 200, 201), comprenant les étapes consistant à :
(a) insérer une pluralité de stents déployables dans un tube de transfert, le tube de transfert comprenant une ou plusieurs fentes ;
(b) déployer les stents partiellement à l'intérieur du tube de transfert à fentes ;
(c) pulvériser un matériau polymère semi-rigide ou rigide à travers la ou les fentes sur une surface externe d'une pluralité d'entretoises des stents ; et
(d) faire durcir la surface externe pulvérisée.

7. Procédé de fabrication selon la revendication 6, dans lequel le durcissement est réalisé à une température et une pression prédéterminées pendant un temps prédéterminé.

8. Procédé de fabrication selon la revendication 6, dans lequel le matériau polymère comprend du THORALON.

9. Procédé de fabrication selon la revendication 6, comprenant en outre l'étape consistant à :
(e) retirer les stents (110, 120, 130) du tube de transfert à fentes.

10. Procédé de fabrication selon la revendication 6, comprenant en outre l'étape consistant à :
(e) créer une zone frangible le long de la surface externe pulvérisée.
